# EUROPEAN PATENT SPECIFICATION

(11) **EP 3 808 751 B1**
(45) Date of publication and mention of the grant of the patent: **26.02.2025**
(21) Application number: 19818832.8
(22) Date of filing: 11.06.2019
(51) Int. Cl.: C07D 491/22, A61K 31/4745, A61P 35/00

(54) **CAMPTOTHECIN DERIVATIVE, PREPARATION METHOD THEREFOR AND APPLICATION THEREOF**
CAMPTOTHECINDERIVAT, HERSTELLUNGSVERFAHREN DAFÜR UND ANWENDUNG DAVON
DÉRIVÉ DE CAMPTOTHÉCINE, PROCÉDÉ DE PRÉPARATION CORRESPONDANT ET UTILISATION ASSOCIÉE

(30) Priority: 12.06.2018 CN 201810600770
(43) Date of publication of application: 21.04.2021
(73) Proprietor: Marine Biomedical Research Institute Of Qingdao Co., Ltd., Qingdao, Shandong 266071 (CN)
(72) Inventor: JIANG, Tao, Qingdao, Shandong 266701 (CN); WU, Guanzhao, Qingdao, Shandong 266701 (CN); HE, Xiong, Qingdao, Shandong 266701 (CN)
(74) Representative: Tiburzi, Andrea
(86) International application number: PCT/CN2019/090731
(87) International publication number: WO 2019/238046

(56) References cited:
- EP-A1- 0 540 099
- WO-A1-2019/195665
- WO-A2-2005/009347
- WO-A2-2005/009347
- WO-A2-2020/061106
- CN-A- 1 074 908
- CN-A- 1 091 742
- US-A- 5 342 947
- US-A- 6 100 273
- US-A1- 2020 231 596
- MANIKUMAR ET AL.: "Camptothecin analogs with bulky, hydrophobic substituents at the 7-position via a Grignard reaction", BIOORG. MED. CHEM. LETT., vol. 14, no. 21, 1 November 2004 (2004-11-01), pages 5377 - 5381, XP004580533, ISSN: 0960-894X, DOI: 10.1016/J.BMCL.2004.08.010
- LACKEY ET AL.: "Water Soluble Inhibitors of Topoisomerase I: Quaternary Salt Derivatives of Camptothecin", J. MED. CHEM., vol. 39, no. 3, 1 January 1996 (1996-01-01), US, pages 713 - 719, XP055887641, ISSN: 0022-2623, DOI: 10.1021/jm950507y
- MANIKUMAR, GOVINDARAJAN: "Camptothecin Analogs with Bulky, Hydrophobic Substituents at the 7-position via a Grignard Reaction", BIOORGANIC & MEDICINAL CHEMISTRY LETTERS, vol. 14, no. 21, 3 September 2004 (2004-09-03), pages 5378 - 5379, XP004580533, ISSN: 0960-894X, DOI: 10.1016/j.bmcl.2004.08.010
- HANSCH, CORWIN: "20-(S)-Camptothecin Analogues as DNA Topoisomerase I Inhibitors: A QSAR Study", CHEMMEDCHEM, vol. 2, no. 12, 20 September 2007 (2007-09-20), pages 1810, XP055668530, ISSN: 1860-7187, DOI: 10.1002/cmdc.200700138
- BACILIERI, MAGDALENA: "A Novel Generalized 3D-QSAR Model of Camptothecin Analogs", MOL. INF., vol. 30, no. 11-12, 1 December 2011 (2011-12-01), pages 930, XP055668537, ISSN: 1868-1751, DOI: 10.1002/minf.201100060

## Description

### Technical field

This invention relates to new camptothecin derivatives and these derivatives for use in the prevention or treatment of cancer.

### Background Art

Camptothecin derivatives are known as one of the three major discoveries of anticancer drugs in 20th century, which showing a broad-spectrum anti-tumor activity and having a huge research and application prospect. For exa mple, the following patent applications or papers disclose several camptothecin d erivatives: WO2019/195665, WO2005/009347, Manikumar et al. Bioorg. Med. Ch em. Lett. 2004, 21, 5377-5381, EO0540099, US6100273, Lackey et al., Med. C hem. 1996, 39, 713-719. Early camptothecin derivatives whose mechanism of ac tion refers to forming a ternary complex with Top I and DNA, thus blocking DNA replication and transcription, and they display low selectivity, high toxicity and some other shortcomings. With the development of technology, molecular-targeting anticancer drugs have became a hotspot in recent years. These drugs can selectively act on signal transduction pathways related to tumor cell differentiation and proliferation. We take camptothecin as a lead compound and modify its structure to develop cand idate compounds with more efficiency and lower toxicity.

The invention provides the novel derivatives which introduce methylenedioxy in the position of 10,11 and different groups in the position of 7, to obtain the compounds which have better antitumor, lower toxicity and better solubleness.

### Summary of Invention

This invention provides a new preparing method and application of camptothecin derivitives, which can be used to prepare drugs for preventing or treating tumors.

To solve the problems, the following techniques are applied. The invention is directed to compounds according to Formula I as well as their stereoisomer, tautomer or pharmaceutically acceptable salt:

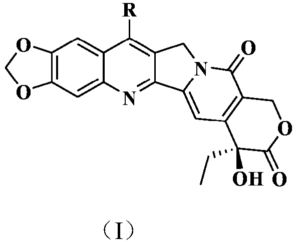

According to an aspect which is not part of the present invention, R could be 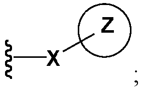
X could be 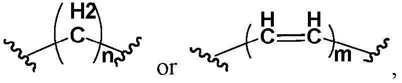 n could be 0, 1 and 2; m could be 0, 1 and 2;
Z could be ring structure; Z is selected from the substituted group or the unsubstituted group of benzene, pyridine, furan, thiophene, pyrazol, benzpyrole, indazole, piperidine, morpholine, thiomorphline , naphthalene or triazole;
When Z is selected from the substituted structures, the substituted groups could be halogen, substituted or unsubstituted of alkyl, substituted or unsubstituted of ester, substituted or unsubstituted of benzene, substituted or unsubstituted of pyrrolidine, substituted or unsubstituted of piperidine, substituted or unsubstituted of morpholine or substituted or unsubstituted of thiomorphline;
The substitution coulde be single substitution or multiple substitution; When n and m are 0, Z could be not unsubstituted benzene.

Preferably, the halogen atom is F.

The stereo isomers including: conformer, optical isomer (such as enantiomorphism isomers and diastereomer), geometric isomers (e.g., cis/trans isomers). The isomer or their combinations can be used as a racemic mixture, separate enantiomorphism isomers, separate diastereomer, diastereomer compounds, cis or trans isomer.

Pharmaceutically acceptable salt refers to pharmaceutically acceptable, organic or inorganic acid or base salt of a compound of the invention. Acceptable salts include but no limited, (1) salts formed with the following inorganic acids: e.g., hydrochloride, hydrobromide, iodide, sulfuric acid, phosphoric acid, nitric acid, (2) salts formed with the following organic acids: e.g., acetic acid, lactic acid, citric acid, succinic acid, fumaric acid, glucose acid, benzoic acid, methane sulfonic acid, ethane sulfonate, benzenesulfonic acid, p-toluene sulfonic acid, oxalic acid, succinic acid, tartaric acid, maleic acid, or arginine, (3) other salts, including salts formed with alkali metal or alkaline earth metal (such as sodium, potassium, calcium or magnesium salt, ammonium salt or water-soluble amine salt (such as N-methyl glucosamine salt), lower alkanol ammonium salt and other pharmaceutical acceptable amine salt (such as methylamine, ethylamine, propylamine, dimethyl amine salt, trimethyl amine salt, triethyl amine salt, tertiary butyl amine salt, ethylenediamine, hydroxylethyl amine salt, morpholine, piperazine, lysine amine salt), or other regular prodrug.

Further the present invention also provides the precursor compounds that are not part of the invention. Precursor described refers to the precursor compound are metabolized or chemically reacted in the patient's body, after being taken by an appropriate method, and then the precursor compounds are converted into general formula, as well as solution or salt of compounds. In this invention precursor compounds described including but not limited to carboxylic acid esters, carbonate, phosphate, nitrate, sulfate, sulfone ester, sulfoxide ester and amino compounds, amino formate, azo compound, phosphoramide, glucoside, ether, acetal and other forms.

In some cases which are not part of the present invention, the compounds mentioned in 1 contain the structure, X could be 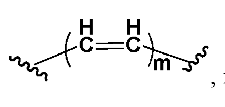 m could be 1 or 2; Z could be substituted or unsubstituted group of benzene, R of the formula I selected optionally from:

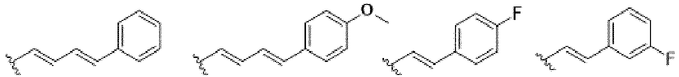

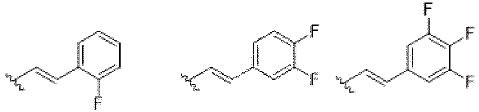

In some cases which are not part of the present invention, X could be 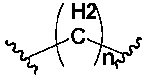 , n could be 2; Z could be substituted or unsubstituted group of benzene, R of the formula I selected optionally from:

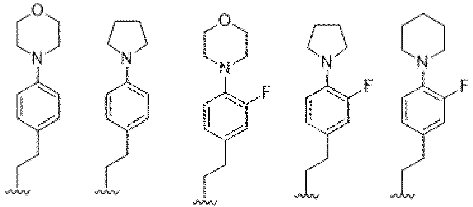

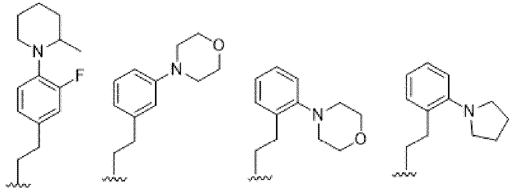

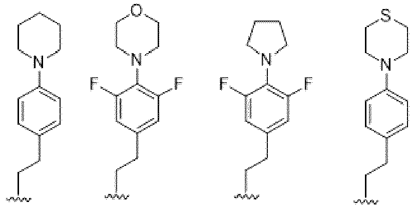

According to the present invention, R of the formula I is selected from:

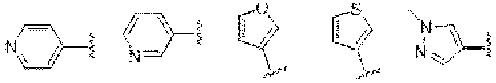

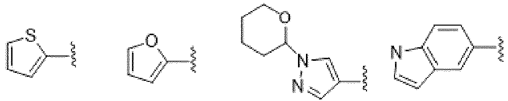

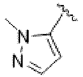

To be specific, the compounds of the invention can be:

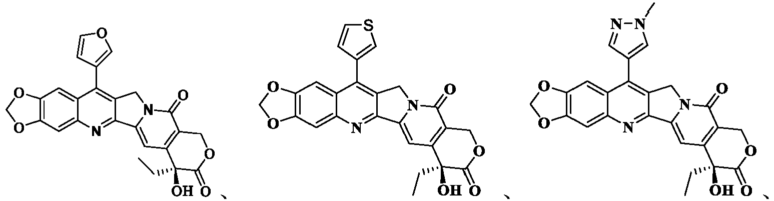

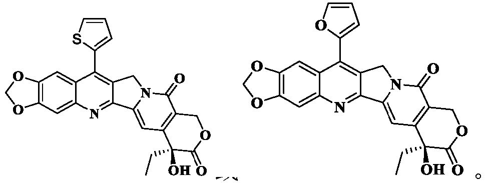

In some cases which are not part of the present invention, X could be 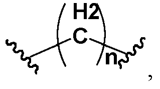 n could be 0,1 and 2; Z selected from substituted or unsubstituted group of piperidine, morpholine or thiomorphline, R of the formula I selected optionally from:

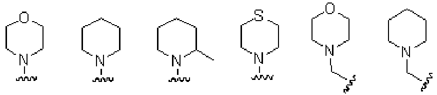

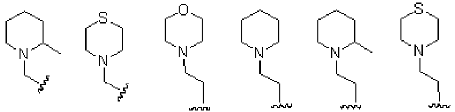

In some cases which are not part of the present invention, R of the formula I could be substituted or unsubstituted group of triazole; the compounds contain the structure of Formula II:

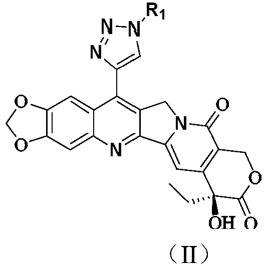

The R₁ could be substituted or unsubstituted group of alkyl, ester, isohydroxamic acid or benzene.

The invention provides the camptothecin derivatives of the invention for use to prevent and/or treat cancer in some extent. Cancer could be lung cancer, prostate cancer, colon cancer, leukemia and breast cancer.

Some compounds could be an inhibitor of EGF or FGF in some extent.

The invention also provides a combination of drugs, including: 1) Effective dose of any compounds in the patent and 2) pharmaceutical acceptable carrier.

The drug combination could be troche, capsule, powder, granule, suppository, oral agents, sterile parenteral suspension and other liquid preparation form and large or small volume injection, lyophilized powder and other injection forms. The forms mentioned can be prepared in a regular synthesis method.

For the invention, drug combination can also join one or more pharmaceutical acceptable carrier, including pharmaceutical field of conventional diluter, filler, adhesive, wetting agents, absorption enhancer, surfactant, adsorption carrier and lubricant, etc

Provided herein is also the synthesis method, which is not part of the invention, of preparing the compounds, and the processes are as follows:
(1) Starting from the raw material of 3,4-methylenedioxyacetophenone (A), 6-amino-3,4-methylenedioxyacetophenone (C) was got through nitration by concentrated nitric acid and hydrogenation by Pd/C. 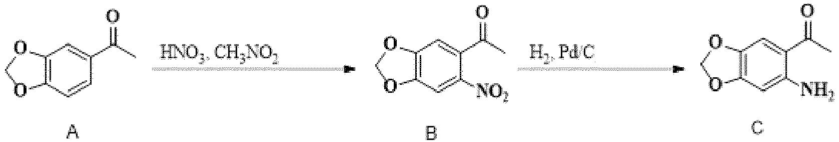
(2) When it comes to formula (I), X is 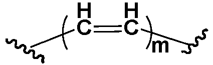 and m is 1or 2; Z i s substituted or unsubstituted group of benzene and R is the group of compoun ds 1-7: 6-amino-3,4-methylenedioxyacetophenone reacted with optionally substitut ed benzaldehyde or cinnamyl aldehyde through a Claisen-Schmidt reaction. The compound we got was 6-amino-3,4-methylenedioxyacetochalcone (D), it reacted with (4S)-4-Ethyl-7,8-dihydro-4-hydroxy-1H-pyrano[3,4-f]indolizine-3,6,10(4H)-trio ne (M) was catalyzed by p-toluenesulfonic acid under reflux for 12hrs, in this way, a Friedlander condensation took place. The compounds were purified by column chromatography and we got the compound 1-7. 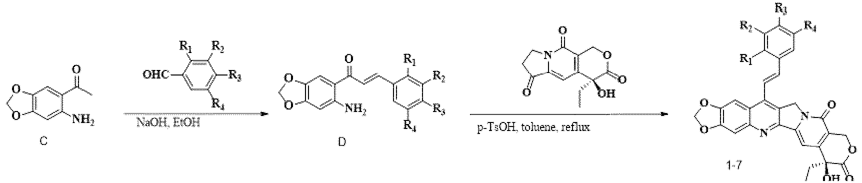
(3) When it comes to formula (I), X is 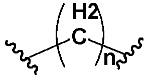 and n is 2; Z is substituted or unsubstituted group of benzene and R is the group of compounds 8-20: the compounds 8-20 were got through hydrogenation of camptothecin der ivatives with styrene in 7 position by Pd/C. 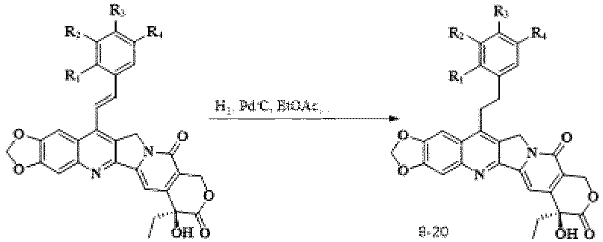
(4) When it comes to formula (I), n and m are 0; R is the group of compound 21-42:
   6-amino-3,4-methylenedioxyacetophenone (C) we got in process of (1) and (4S)-4-Ethyl-7,8-dihydro-4-hydroxy-1H-pyrano[3,4-f]indolizine-3,6,10(4H)-trione (M) too k condensation reaction in the present of p-toluenesulfonic acid under reflux. The n acylation with acetic acid and oxidiziing reaction with hydrogen peroxide wer e took place to get compound F. Compound F reacted with oxalyl bromide to obtain 20(S)-O-acetyl-7-chlorine-10,11-methylenedioxy-camptothecin (G). The com pounds 21-42 were got through the coupling action by G and substituted phenyl group or alkenylboronic acids, and remove the acetyl group by sodium methoxide. 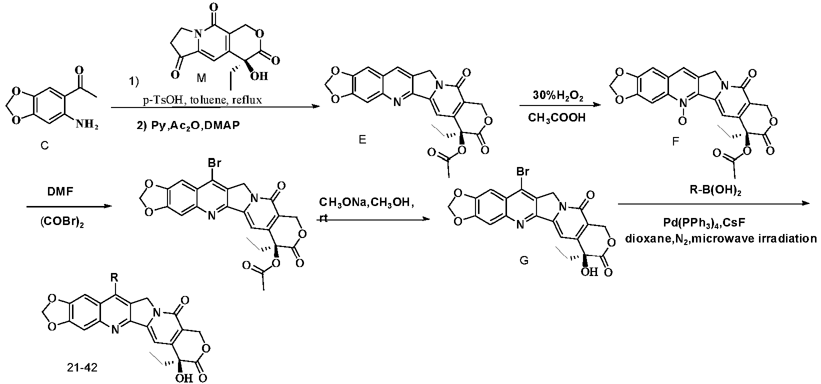
(5) When it comes to formula (I), R is the group of compounds 43-46: the com pound 20(S)-O-acetyl-7-chlorine-10,11-methylenedioxy-camptothecin (G) from pro cess (4) reacted with different heterocyclic and then got the compounds 43-46 which we re substituted by N-methyl morpholine, N-methyl piperidine, N-methyl-2-methyl piperidine and N-methyl thiomorpholine at 7th position.
(6) When it comes to formula (I), R is the group of compounds 47-50: the com pound F from process (4) reacted with nitrogen heterocyclic and got the compounds 47-5 0 which were substituted by N-methyl morpholine, N-methyl piperidine, N-meth yl-2-methyl piperidine and N-methyl morpholine sulfur generation at 7th position.
(7) When it comes to formula (I), R is the group of compounds 51-54: the com pound J was got through reacted with compound C, condensation by N,N-Dimethylforma mide dimethyl acetal, substitutation by heterocyclic group, then these compounds reacted with (4S)-4-Ethyl-7,8-dihydro-4- hydroxy-1H-pyrano[3,4-f]indolizine-3,6,1 0(4H)-trione (M) catalyzed by p-toluenesulfonic acid under reflux to get compounds 51-54 which were substituted by Morpholine ethyl, Thiomorpholine ethyl, 2-Methylpiperidineethyl at 7th position.
(8) when it comes to formula (II), of which the 7th position of 10,11 -ethylenedi oxy-camptothecin is triazole substituted by different substituent groups, the preparing process is as follows: 20(S)-O-acetyl-7-chlorine-10,11-methylenedioxy-c amptothecin reacted with trimethylsilylaceyene under palladiumacetate to afford trimethylsilylaceyene group on the 7th position, and it reacted with potassium fluoride, then we got 20(S)-O-acetenyl-7-chlorine-10,11-methylenedioxy -camptothecin which reacted with azides substituted by different substituent groups. The compounds were purified by column chromatography and we got 10,11-methylenedioxy-camptothecin derivitives with 7th position substituted by different triazole substituent groups.

Provided herein is also a preparing method of intermediates (M), which is not part of the invention:

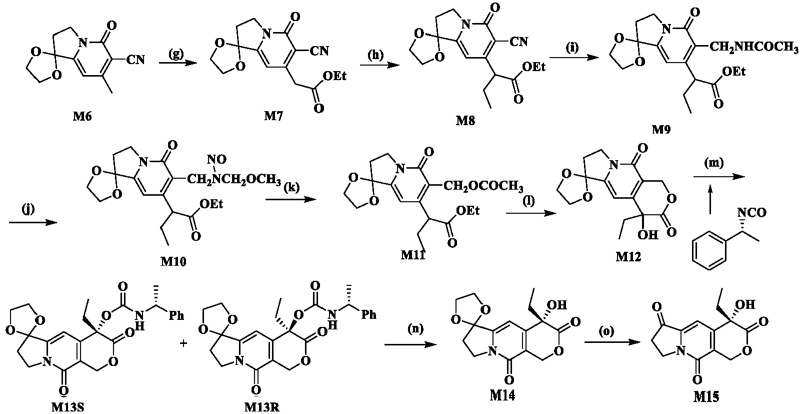

Compound M6 reacted with diethyl carbonate at the present of potassium tert-butoxide to yield intermediate M7; M9 was got by reacting with bromoethane and then hydrogenation by Ni/H₂; M9 reacted with sodium nitrite to get M10 and then through rearrangement in CCl₄ and oxidization to obtain M 12 which was a chiral compound. Chiral isocyanates was used to react with M 12 catalyzed by copper chloride and we can get two non-corresponding isomers through column chromatography; M13S was hydrolyzed by base and deprotected by TFA to obtain M15.

Compounds of the present invention showed better selectivity to tumor and better solubility than traditional camptothecin compounds. The antitumor activity of the compounds was increased compared with the parent compound significantly, while maintaining the low toxicity. They can be used for the preparation of prevention or treatment of cancer drugs. Camptothecin derivatives in this invention have clear structural features, easy to synthesis and purified, with good biological activity. So this kind of compounds in the prevention or treatment of cancer has a broad application prospect.

### Description of Drawings

Figure 1.Synthesis scheme of the compounds in this invention.

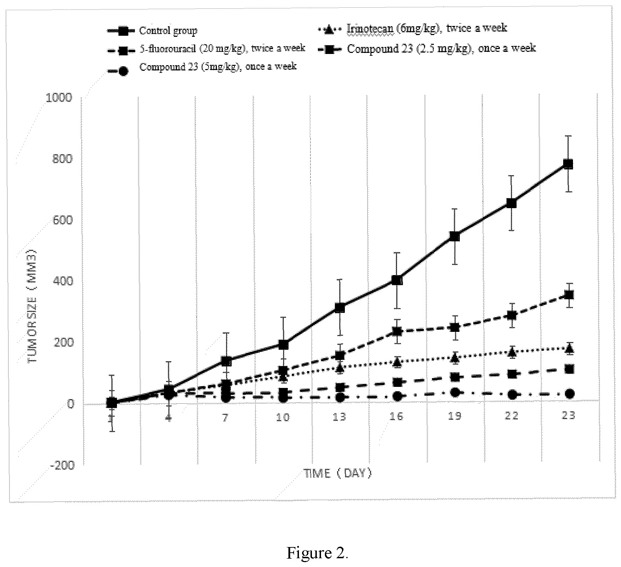

Figure 2.Efficient of compound 23 on tumor volume change in HCT-116 transplanted tumor model mice.
Figure 3.Efficient of compound 23 on weight change in HCT-116 transplanted tumor model mice.

### Detailed description of the invention

Method and technology in this invention are generally based on the known traditional methods in the field, unless claimed specially. The description in this article of the nomenclature in the field of biology, pharmacology, and medical and pharmaceutical chemical, and the experimental method and technique are known and used in this field. Chemical synthesis, chemical analysis, medical method, mixing method and transmission method, as well as the patient's treatment adopt standard technique.

Unless claimed specially, the science and technical terms used in this article should have the common meaning of this field. But the following term has the following definition:
"Cancer" refers to a variety of diseases with abnormal cells in the body characterized by uncontrolled growth. Uncontrolled cell division and growth division lead to a vicious tumor or cell. They invade neighboring tissues and can also transferred to the distal parts of the body through the lymphatic system or bloodstream. "Treat cancer" in the present invention is another equivalent description "treat tumor" or "anticancer", or "antitumor".

Cancer is the diseases which uncontrolled cells interfere with the normal function of the body organs and systems. Cancer of the subjects is objectively exists in the subjects that can be measured by the subjects of cancer cells. The subjects at risk of developing cancer are easy to develop cancer (based on family history, genetic predisposition, for example) of the subjects, exposure to radiation or other reagents.

The compounds in invention can be used to treat a variety of subjects of cancer or at risk of developing cancer. Examples of such cancers including breast, prostate cancer, lung cancer, ovarian cancer, cervical cancer, skin cancer, melanoma, colon cancer, gastric cancer, liver cancer, esophageal cancer, kidney cancer, throat cancer, thyroid cancer, pancreatic cancer, testicular cancer, brain cancer, bone cancer and leukemia (such as leukemia, chronic lymphocytic leukemia), etc. Other cancers including but not limited to, basal cell carcinoma, billiard tract cancer, bladder cancer, bone cancer, brain and central nervous system (CNS) cancer, cervical cancer, choriocarcinoma, connective tissue, cancer, digestive system cancer, colorectal cancer, endometrial carcinoma, esophageal cancer, eye cancer, head and neck cancer, epithelial tumors, laryngeal cancer, lung cancer, small cell, large cells), lymphoma (including Hodgkin's lymphoma and non-hodgkin's lymphoma); melanoma; neuroblastoma. Oral cancer (such as lips, tongue, mouth and throat); retinoblastoma.; leiomyosarcoma; respiratory cancer; sarcoma; ovarian cancer; urinary tract cancer; as well as other carcinoma and sarcoma.

"Effective dose" are drugs used as described below, when used alone or combined with another therapeutic agent using the amount of drugs, can promote disease subsided that the symptoms of disease severity, the frequency of no disease symptoms and duration increased, or prevent disorder caused by illness or disability.

Drug treatment effective dose or dose including "preventive effective dose" or "prevention effective dose", "prevention effective dose" or "prevention effective dose is the amount of drugs mentioned in any of the following, when the amount of drug use alone or combined with another therapeutic agent applied to the risk of disease or suffered from relapse of subjects, can inhibit the occurrence or recurrence of disease. Agents to promote disease fade or inhibit the ability of development or recurrence can use technical staff known to evaluate the various methods, such as in human subjects in clinical trials, in predictable in the effectiveness of animal models of human system, or by in vitro activity of measuring system was developed for the determination of reagent.

As an example, anticancer agent (drug combination for the treatment of cancer) promotes the subjects of tumor regression in the body. The implementation of the scheme, the optimal selection of effective dose of the drug to promote cancer cells fades or even eliminate the cancer. "Promote cancer resolve" means to be used alone or in combination with antitumor agent (anti - neoplastic agent) combination use of lead to the growth of tumors by effective dose of drug treatment or size reduction, tumor necrosis, at least one disease symptom is reduced, and the severity of the disease free frequency and duration of symptoms phase increases, prevent disorder caused by illness or disability, or in other ways to improve the patient's disease symptoms.

In addition, the terms about the treatment of "efficient" and "effectiveness" include security both effectiveness of pharmacology and physiology. Pharmacology effectiveness is the ability to drugs promote cancer patients subsided. Physiology security refers to the cells and organs due to drug use and/or biological level of toxic or other harmful physiological effects, adverse effects).

As an example of tumor treatment, compared with the untreated subjects, effective dose and dose of the drug treatment optimization can inhibit the growth of cells or tumor growth of about 20%, at least preferred at least about 40%, or even more preferred at least about 60%, and also preferred at least about 80%. In the optimization of the implementation of the scheme, effective dose and dose of the drug treatment can completely inhibit the growth of cells or tumor growth, namely optimizing to inhibit cell growth or tumor growth of 100%. Compounds that inhibit the ability of tumor growth can be evaluated in the animal model. They are able to predict efficacy of tumor. Instead, the combination of this nature can be evaluated by taking a test that compounds inhibiting the growth of cells. Such inhibition of this can be measured by technical staff in a known measurement in vitro. In other optimization scheme of the invention, the tumor regression can be observed, and lasts at least 20 days, preferred at least 40 days, or even preferred at least 60 days.

The subject "treatment" refers to reversing, reducing and treating, inhibiting, slowing or preventing any related to disease symptoms, complications, illness, or the occurrence of biochemical indicators, progress, development, severity or recurrence for the purpose to make any type of intervention in subjects.

Provided herein are the camptothecin derivatives shown in the table below. Only the compounds 21-30 are according to the invention. The compounds 1-20 and 31-55 are reference examples and not part of the invention.

| No. | Structure | NO. | Structure |
|---|---|---|---|
| 1 | 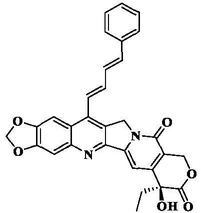 | 29 | 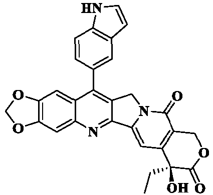 |
| 2 | 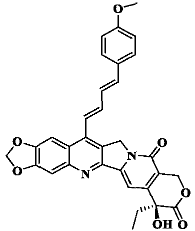 | 30 | 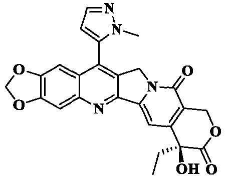 |
| 3 | 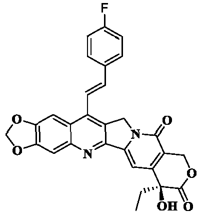 | 31 | 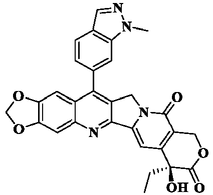 |
| 4 | 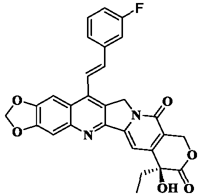 | 32 | 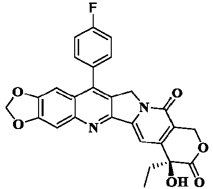 |
| 5 | 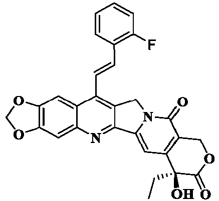 | 33 | 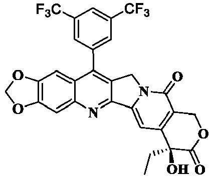 |
| 6 | 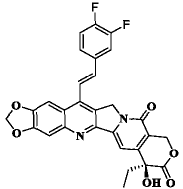 | 34 | 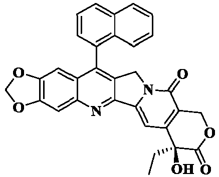 |
| 7 | 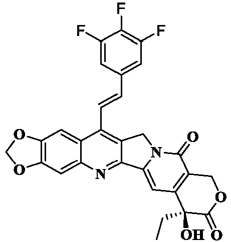 | 35 | 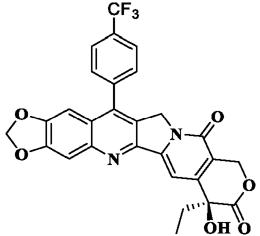 |
| 8 | 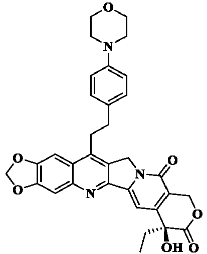 | 36 | 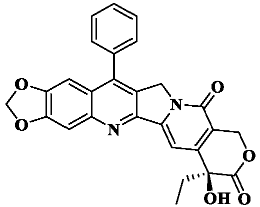 |
| 9 | 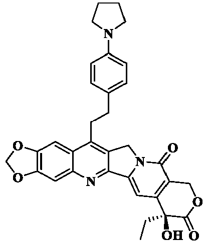 | 37 | 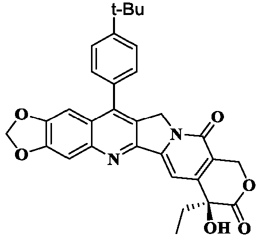 |
| 10 | 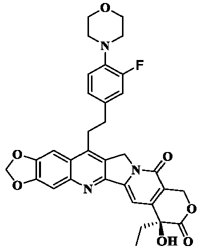 | 38 | 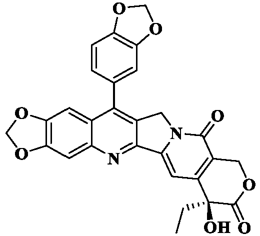 |
| 11 | 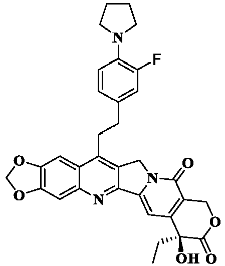 | 39 | 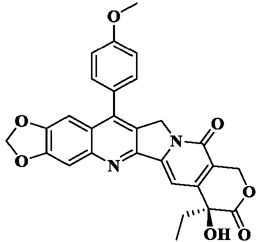 |
| 12 | 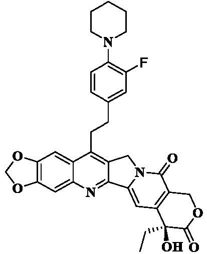 | 40 | 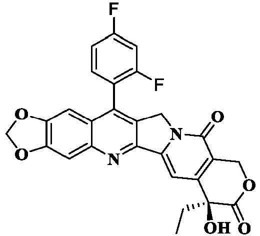 |
| 13 | 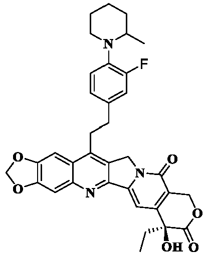 | 41 | 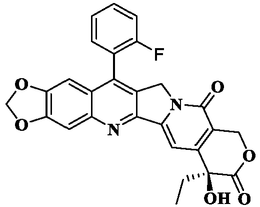 |
| 14 | 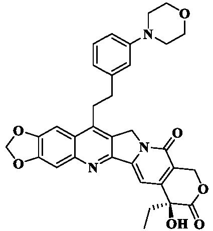 | 42 | 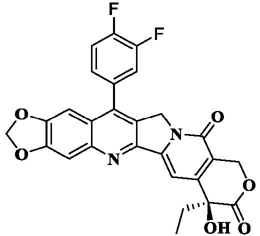 |
| 15 | 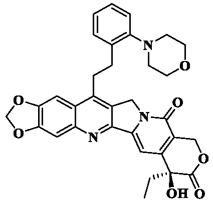 | 43 | 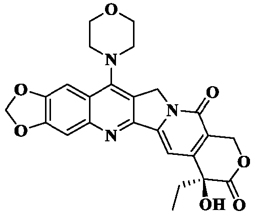 |
| 16 | 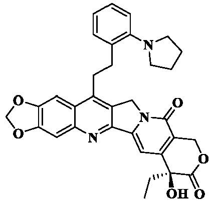 | 44 | 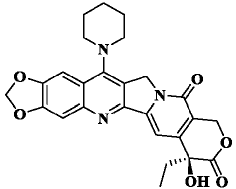 |
| 17 | 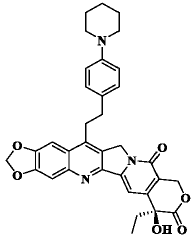 | 45 | 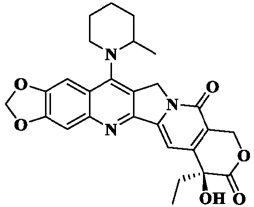 |
| 18 | 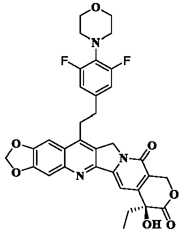 | 46 | 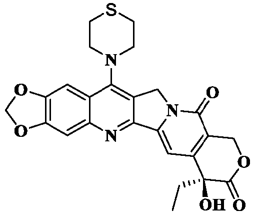 |
| 19 | 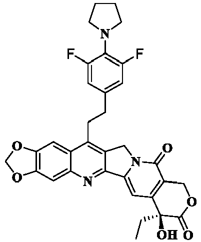 | 47 | 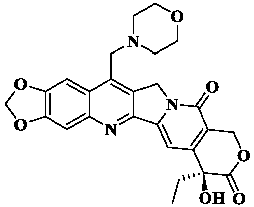 |
| 20 | 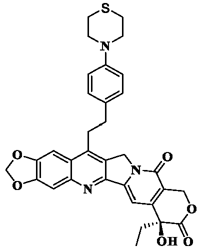 | 48 | 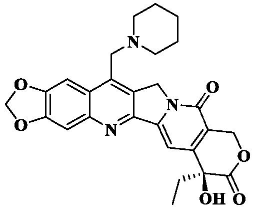 |
| 21 | 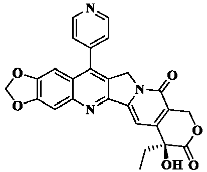 | 49 | 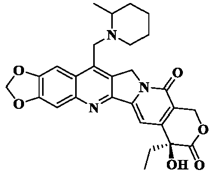 |
| 22 | 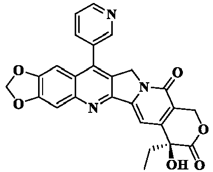 | 50 | 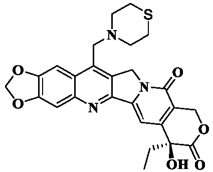 |
| 23 | 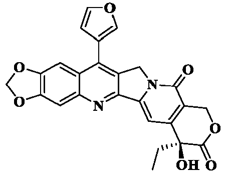 | 51 | 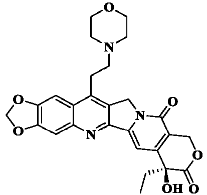 |
| 24 | 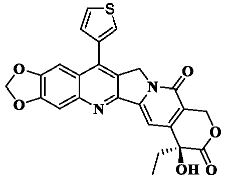 | 52 | 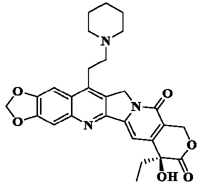 |
| 25 | 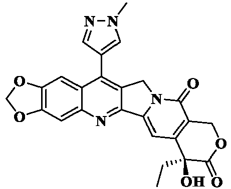 | 53 | 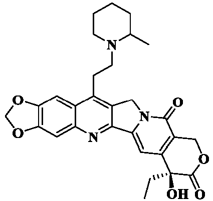 |
| 26 | 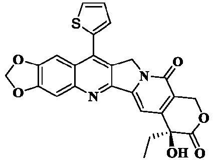 | 54 | 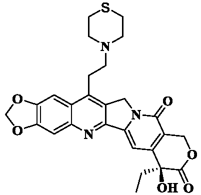 |
| 27 | 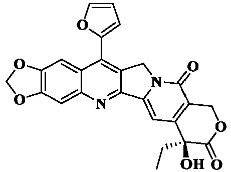 | 55 | 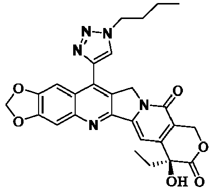 |
| 28 | 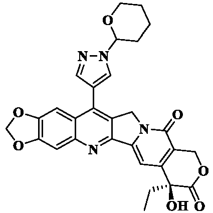 | | |

After the test, the present invention compounds synthesized compounds are generally have better solubility in water.

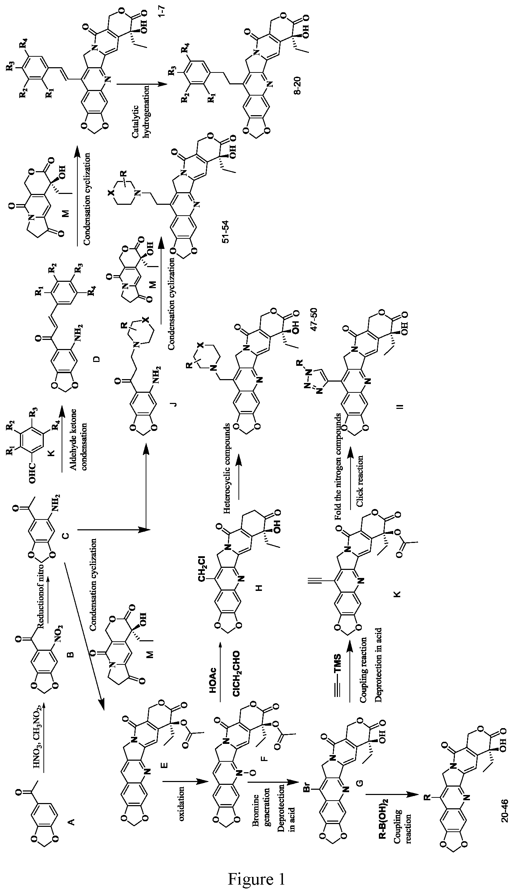

Figure 1 shows the above compound synthetic route diagram. Combined with concrete example to illustrate the implementation of the below:
The synthesis process of compounds 1-7 was as following:

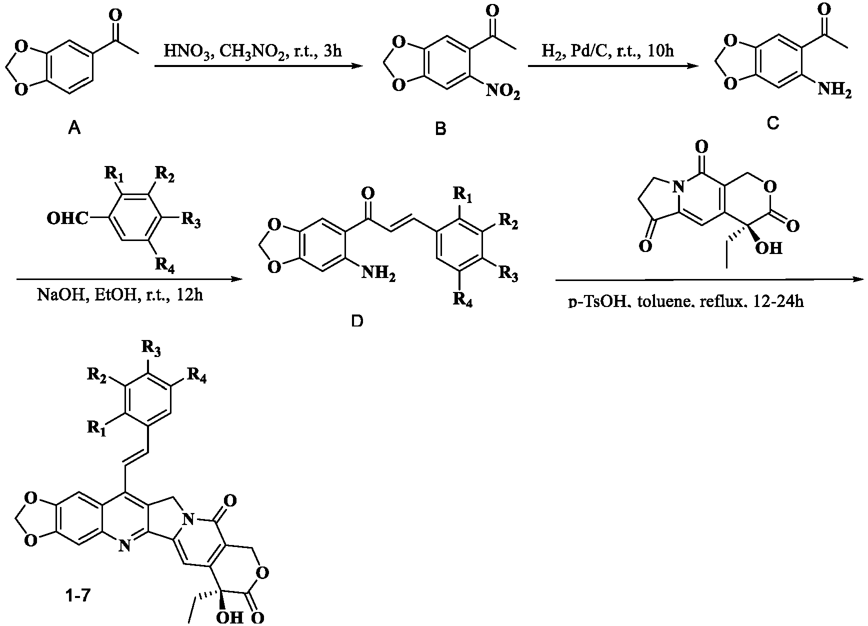

### Reference Example 1 20(S)-7-(styrene-butadiene-yl)-10,11-methylenedioxy-camptothecin (compound 1)

### 1) preparation of (E)-1-(6-aminobenzo[d][1,3]dioxol-5-yl)-3-phenylprop-2-en-1-one (D1):

Concentrated nitric acid (20.7mL) was added dropwise to the solution of 3,4-methylenedioxy acetophenone was dissolved in nitromethane (80mL), and the mixture reacted for 2 hrs. Then saturated sodium bicarbonate solution was added dropwise to the reaction mixture to get the pH to about 7. The mixture was extracted with dichloromethane for 3 times. The organic layer was combined, washed with brine, dried by anhydrous MgSO₄ and concentrated, then purified by column chromatography (ethyl acetate: petroleum ether=1:8) to afford 6-nitro-3,4-methylenedioxy acetophenone, light yellow solid, yield: 7.8 g (76%). mp 110-112°C.

6-nitro-3,4-methylenedioxy acetophenone (7.8g, 37.3mmol) was dissolved in ethyl acetate(70mL) and catalytic amount Pd/C was added. The mixture reacted under hydrogen condition for 12hrs. The solution was filtered and the filtrate was concentrated to afford 6-amino-3,4-methylenedioxy acetophenone, light yellow solid, yield: 5.5 g (83%). mp 123-124°C.

Sodium hydroxide (0.4g, 11mmol) and benzaldehyde(0.14g, 1.3mmol) were added to the solution of 6-amino-3,4-methylenedioxy acetophenone (0.2g, 1.1mmol) in anhydrous alcohol (10mL). The mixture reacted for 12hrs under room temperature and solution was concentrated. The mixture was purified by column chromatography (ethyl acetate: petroleum ether = 1:4) to afford D1, yellow solid, yeild: 0.21 g (71%). mp 130-132°C.

¹H NMR (CDCl₃, 600 MHz) δ: 8.08 (s, 1 H, ArH), 7.81 (d, *J* =15.2 Hz, 1 H, =CHAr), 7.53 (d, *J* =15.2 Hz, 1 H, ArCOCH=), 6.79 (d, *J* =7.7 Hz, 2 H, ArH), 6.67 (d, *J =*7.7 Hz, 2 H, ArH), 6.60 (t, *J* =7.7 Hz, 1 H, ArH), 6.54 (s, 1 H, ArH), 6.20 (brs, 2 H, NH₂), 6.02 (s, 2 H, OCH₂O).¹³C NMR (126 MHz, DMSO-d₆) δ 192.0, 164.9, 163.0, 152.7, 149.3, 144.6, 141.4, 132.7, 131.6, 131.1, 126.3, 116.5, 116.3, 108.1, 105.3, 104.5.

### 2) preparation of 20(S)-7-(styrene-butadiene-yl)-10,11-methylenedioxy-camptothecin (compound 1)

(E)-1-(6-aminobenzo[d][1,3]dioxol-5-yl)-3-phenylprop-2-en-1-one (D1) (200 mg, 0.76 mmol) was dissolved in anhydrous toluene (70 mL)., and then (S)-4-ethyl-4-hydroxy-7,8-dihydro-1H-pyrano[3,4-f]indolizine-3,6,10(4H)-trione (0.41 g, 1.4 mmol) and *p*-toluenesulfonic acid (13.0 mg, 0.08 mmol) were added. The mixture reacted at 110°C under nitron condition for 24 hrs. The mixture was cooled to room temperature and the reaction mixture was concentrated, then purified by column chromatography (dichloromethane: acetone = 10:1) to afford compound 1, light yellow solid, yeild: 60 mg (15%). mp 206-214 °C; MS(ESI): m/z, 521.4 [M+H]⁺.

¹H NMR (600 MHz, CF₃COOD) δ 8.23 (s, 1H), 8.06 - 7.94 (m, 1H), 7.92 (s, 1H), 7.84 (d, *J* = 16.4 Hz, 1H), 7.79 (s, 1H), 7.72 (d, *J =* 7.4 Hz, 1H), 7.67 (d, *J =* 18.5 Hz, 1H), 7.53 (d, *J =* 8.1 Hz, 1H), 7.48 (d, *J =* 15.6 Hz, 1H), 7.43 (d, *J =* 9.7 Hz, 1H), 7.36 (s, 2H), 7.29 - 7.14 (m, 1H), 6.57 - 6.43 (m, 2H), 6.01 (d, *J =* 26.7 Hz, 2H), 5.70 (d, *J* = 16.7 Hz, 1H), 2.29 (d, *J =* 27.2 Hz, 2H), 1.35 (dd, *J =* 117.8, 12.9 Hz, 3H). ¹³C NMR (151 MHz, CF₃COOD) δ 176.5, 161.1, 157.5, 157.6, 149.3, 147.4, 146.5, 145.5, 134.3, 131.7, 130.3, 129.5, 128.7, 128.5, 128.0, 127.9, 127.4, 126.9, 121.0, 117.9, 115.7, 113.8, 111.2, 105.5, 97.4, 74.0, 66.5, 65.5, 31.7, 11.8, 5.4.

### Reference Example 2 20(S)-7-(4-fluorostyryl)-10,11-methylenedioxy-camptothecin (compo und 3)

The synthesis and purification of Compound **3** was described as compound 1, only acetophenone was substituted by 4-fluoro benzaldehyde. mp >250°C; MS(ESI): m/z, 513.5 [M+H]⁺.

¹H NMR (500 MHz, CF₃COOD) δ 8.23 (s, 1H), 8.00 (s, 1H), 7.95 (dd, *J =* 7.8, 5.4 Hz, 1H), 7.87 (d, *J =* 16.5 Hz, 1H), 7.85 - 7.78 (m, 1H), 7.73 (s, 1H), 7.30 (dd, *J =* 22.8, 14.3 Hz, 2H), 7.10 (dd, *J =* 36.5, 25.0 Hz, 1H), 6.52 (s, 2H), 6.03 (t, *J =* 11.9 Hz, 1H), 6.01 - 5.83 (m, 2H), 5.69 (d, *J =* 17.0 Hz, 1H), 2.24 (dd, *J =* 17.8, 11.0 Hz, 2H), 1.33 - 1.16 (m, 3H).

¹³C NMR (125 MHz, CF₃COOD) δ 176.2, 157.4, 152.1, 151.8, 148.9, 145.0, 140.1, 139.8, 138.8, 130.6, 130.0, 130.0, 126.1, 126.0, 121.0, 117.3, 116.1, 116.0, 105.0, 103.4, 101.1, 97.3, 73.9, 66.2, 52.5, 31.0, 11.7, 5.8.

### Reference Example 3 20(S)-7-(3-fluorostyryl)-10,11-methylenedioxy-camptothecin (compo und 4)

The synthesis and purification of Compound **4** was described as compound 1, only acetophenone was substituted by 3-fluoro benzaldehyde. mp >250°C; MS(ESI): m/z, 513.5 [M+H]⁺.

¹H NMR (500 MHz, CF₃COOD) δ 8.23 (s, 1H), 8.00 (s, 1H), 7.95 (dd, *J =* 7.8, 5.4 Hz, 1H), 7.87 (d, *J =* 16.5 Hz, 1H), 7.85 - 7.78 (m, 1H), 7.73 (s, 1H), 7.30 (dd, *J =* 22.8, 14.3 Hz, 2H), 7.14 (dd, *J =* 36.5, 25.0 Hz, 1H), 6.56 (s, 2H), 6.03 (t, *J =* 11.9 Hz, 1H), 6.21 - 5.84 (m, 2H), 5.69 (d, *J =* 17.0 Hz, 1H), 2.24 (dd, *J =* 17.8, 11.0 Hz, 2H), 1.33 - 1.16 (m, 3H).

¹³C NMR (126 MHz, CF₃COOD) δ 176.2, 157.4, 152.1, 151.8, 148.9, 145.0, 140.1, 139.8, 138.8, 130.6, 130.4, 130.0, 126.8, 126.0, 121.0, 117.5, 116.1, 116.0, 105.0, 103.4, 101.1, 97.3, 73.9, 66.2, 52.5, 31.0, 11.7, 5.8.

### Reference Example 4 20(S)-7-(2-fluorostyryl)-10,11-methylenedioxy-camptothecin (compo und 5)

The synthesis and purification of Compound **3** was described as compound 1, only acetophenone was substituted by 3-fluoro benzaldehyde. mp >250°C; MS(ESI): m/z, 513.5 [M+H]⁺.

¹H NMR (500 MHz, CF₃COOD) δ 8.23 (s, 1H), 8.00 (s, 1H), 7.95 (dd, *J =* 7.8, 5.4 Hz, 1H), 7.87 (d, *J =* 16.5 Hz, 1H), 7.81 - 7.78 (m, 1H), 7.53 (s, 1H), 7.30 (dd, *J =* 22.8, 14.3 Hz, 2H), 7.28 (dd, *J =* 36.5, 25.0 Hz, 1H), 6.56 (s, 2H), 6.03 (t, *J =* 11.9 Hz, 1H), 6.21 - 5.84 (m, 2H), 5.69 (d, *J =* 17.0 Hz, 1H), 2.24 (dd, *J =* 17.8, 11.0 Hz, 2H), 1.33 - 1.16 (m, 3H).

¹³C NMR (126 MHz, CF₃COOD) δ 176.2, 157.4, 152.1, 151.8, 148.9, 145.0, 140.1, 139.7, 138.8, 130.9, 130.4, 130.0, 126.8, 126.0, 121.0, 117.5, 116.1, 116.0, 105.0, 103.4, 101.1, 97.3, 73.9, 66.2, 52.5, 31.0, 11.7, 5.8.

The synthesis scheme of **8-20** was as follows:

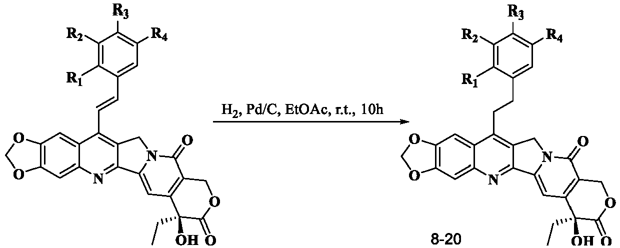

### Reference Example 5 20(S)-7-(4-(4-morpholino)phenethyl)-10,11-methylenedioxy-camptoth ecin (compound 8)

(S,E)-7-ethyl-7-hydroxy-14-(4-morpholinostyryl)-10,13-dihydro-11H-[1,3]dioxol o[4,5-g]pyrano[3',4':6,7]indolizino[1,2-b]quinoline-8,11(7H)-dione was prepared and purified as described for the synthesis and purifying of compound 1 using 4-morpholine benzaldehyde and then dissolved in EA at the present of Pb/C under hydrogen for 10 hrs. The mixture was filtered and the filtrate was concentrated, then purified by column chromatography (dichloromethane: acetone = 10:1) to afford compound 8. mp >250°C; MS(ESI): m/z, 513.5 [M+H]⁺.

¹H NMR (500 MHz, DMSO) δ 7.58 (s, 1H), 7.46 (s, 1H), 7.19 (s, 1H), 6.95 (d, *J* = 8.3 Hz, 2H), 6.74 (d, *J =* 8.1 Hz, 2H), 6.25 (s, 2H), 5.33 (t, *J =* 11.8 Hz, 2H), 4.75 (q, *J* = 18.8 Hz, 2H), 3.65 (s, 4H), 3.26 (s, 2H), 2.93 (s, 4H), 2.83 (s, 2H), 1.87 - 1.76 (m, 2H), 0.83 (t, *J =* 7.2 Hz, 3H).

¹³C NMR (125 MHz, DMSO) δ 172.94, 157.14, 151.29, 150.49, 150.10, 149.44, 149.38, 147.54, 146.67, 142.38, 131.83, 129.60, 128.32, 124.68, 118.31, 115.57, 105.88, 103.01, 99.94, 96.45, 72.74, 66.40, 65.63, 49.85, 49.22, 34.39, 32.34, 30.62, 8.17.

### Reference Example 6 20(S)-7-(4-(pyrrolidin-1-yl)phenethyl)-10,11-methylenedioxy-camptot hecin (compound 9)

(S,E)-7-ethyl-7-hydroxy-14-(4-(pyrrolidin-1-yl)styryl)-10,13-dihydro-11H-[1,3]di oxolo[4,5-g]pyrano[3',4':6,7]indolizino[1,2-b]quinoline-8,11(7H)-dione was prepared and purified as described for the synthesis and purifying of compound 1 using 4-pyrrolidin benzaldehyde and then dissolved in EA at the present of Pb/C under hydrogen condition for 10 hrs. The mixture was filtered and the filtrate was concentrated, then purified by column chromatography (dichloromethane: acetone = 10:1) to afford compound 9; mp 205-207°C.

¹H NMR (500 MHz, DMSO) δ 7.61 (s, 1H), 7.47 (s, 1H), 7.19 (s, 1H), 6.89 (d, *J* = 8.2 Hz, 2H), 6.35 (d, *J =* 8.1 Hz, 2H), 6.26 (s, 2H), 5.35 (s, 2H), 4.77 (q, *J =* 18.7 Hz, 2H), 3.25 (d, *J =* 7.4 Hz, 2H), 3.06 (s, 4H), 2.80 (s, 2H), 1.91 - 1.76 (m, 6H), 0.83 (t, *J* = 7.3 Hz, 3H).

¹³C NMR (125 MHz, DMSO) δ 172.94, 157.12, 151.28, 150.50, 149.47, 149.38, 147.56, 147.07, 146.72, 142.52, 129.54, 128.35, 127.61, 124.68, 118.29, 112.07, 105.93, 103.01, 99.99, 96.37, 72.75, 65.62, 49.88, 47.93, 34.53, 32.71, 30.56, 25.24, 8.18.

### Reference Example 7 20(S)-7-(3-fluoro-4-(4-morpholino)phenethyl)-10,11-methylenedioxy-camptothecin (compound 10)

(S,E)-7-ethyl-14-(3-fluoro-4-morpholinostyryl)-7-hydroxy-10,13-dihydro-11H-[1, 3]dioxolo[4,5-g]pyrano[3',4':6,7]indolizino[1,2-b]quinoline-8,11(7H)-dione was prepared and purified as described for the synthesis and purifying of compound 1 using 3-fluoro-4-(4-morpholine)-benzaldehyde and then dissolved in EA at the present of Pb/C under hydrogen condition for 10 hrs. The mixture was filtered and the filtrate was concentrated, then purified by column chromatography (dichloromethane: acetone = 10:1) to afford compound 10; mp >250°C.

¹H NMR (500 MHz, DMSO) δ 7.56 (s, 1H), 7.44 (s, 1H), 7.20 (s, 1H), 6.99 (d, *J* = 13.3 Hz, 1H), 6.91 - 6.82 (m, 2H), 6.23 (s, 2H), 5.35 (q, *J =* 16.3 Hz, 2H), 4.90 (q, *J* = 18.7 Hz, 2H), 3.66 (s, 4H), 3.27 (s, 2H), 2.84 (s, 6H), 1.81 (dt, *J =* 24.6, 7.0 Hz, 2H), 0.83 (t, *J =* 7.2 Hz, 3H).

¹³C NMR (125 MHz, DMSO) δ 172.98, 157.24, 151.31, 150.56, 149.53, 149.40, 147.54, 146.67, 142.16, 136.18, 128.15, 125.36, 124.72, 119.24, 118.35, 116.75, 116.59, 105.81, 103.02, 99.99, 96.56, 72.77, 66.54, 65.65, 51.07, 49.91, 34.12, 31.77, 30.64, 8.16.

### Reference Example 8 20(S)-7-(3-fluoro-4-(pyrrolidin-1-yl))-10,11-methylenedioxy-camptoth ecin (compound 11)

(S,E)-7-ethyl-14-(3-fluoro-4-(pyrrolidin-1-yl)styryl)-7-hydroxy-10,13-dihydro-11 H-[1,3]dioxolo[4,5-g]pyrano[3',4':6,7]indolizino[1,2-b]quinoline-8,11(7H)-dione was prepared and purified as described for the synthesis and purifying of compound 1 using 3-fluoro-4-(1-pyrrolidin)-benzaldehyde and then dissolved in EA at the present of Pb/C under hydrogen condition for 10 hrs. The mixture was filtered and the filtrate was concentrated, then purified by column chromatography (dichloromethane: acetone = 10:1) to afford compound 11; mp >250°C.

¹H NMR (600 MHz, DMSO-D6) δ 7.60 (s, 1H), 7.47 (s, 1H), 7.23 (s, 1H), 6.89 (dd, *J =* 14.9, 1.9 Hz, 1H), 6.75 (d, *J =* 8.2 Hz, 1H), 6.59 - 6.53 (m, 1H), 6.26 (d, *J =* 2.2 Hz, 2H), 5.37 (q, *J =* 16.0 Hz, 2H), 4.86 (q, *J =* 18.6 Hz, 2H), 3.27 (d, *J* = 6.1 Hz, 2H), 3.15 (d, *J =* 2.1 Hz, 4H), 2.82 (d, *J =* 5.0 Hz, 2H), 1.87 - 1.78 (m, 6H), 0.85 (t, *J =* 7.3 Hz, 3H).

¹³C NMR (151 MHz, DMSO-D6) δ 173.14, 157.34, 151.44, 150.71, 149.53, 149.51, 147.62, 146.78, 142.40, 136.22, 130.86, 128.28, 125.27, 124.79, 118.43, 116.50, 115.97, 105.91, 103.16, 100.04, 96.70, 72.90, 65.74, 50.12, 49.97, 34.11, 32.25, 30.73, 25.07, 8.26.

### Reference Example 9 20(S)-7-(3-fluoro-4-(piperidin-1-yl)phenethyl)-10,11-methylenedioxy-camptothecin (compound 12)

(S,E)-7-ethyl-14-(3-fluoro-4-(piperidin-1-yl)styryl)-7-hydroxy-10,13-dihydro-11H-[1,3]dioxolo[4,5-g]pyrano[3',4':6,7]indolizino[1,2-b]quinoline-8,11(7H)-dione was prepared and purified as described for the synthesis and purifying of compound 1 using 3-fluoro-4-(1-piperidin)-benzaldehyde and then dissolved in EA at the present of Pb/C under hydrogen condition for 10 hrs. The mixture was filtered and the filtrate was concentrated, then purified by column chromatography (dichloromethane: acetone = 10:1) to afford compound 12; mp >250°C.

¹H NMR (500 MHz, DMSO) δ 7.61 (s, 1H), 7.47 (s, 1H), 7.20 (s, 1H), 6.95 (d, *J* = 13.4 Hz, 1H), 6.86 - 6.80 (m, 2H), 6.25 (s, 2H), 5.34 (t, *J =* 9.8 Hz, 2H), 4.90 (t, *J =* 13.9 Hz, 2H), 3.30 (s, 2H), 2.82 (d, *J =* 25.5 Hz, 6H), 1.82 (td, *J =* 14.2, 6.7 Hz, 2H), 1.56 (s, 4H), 1.45 (s, 2H), 0.83 (t, *J =* 7.3 Hz, 3H).

¹³C NMR (125 MHz, DMSO) δ 172.95, 157.19, 151.30, 150.48, 149.56, 149.40, 147.58, 146.69, 142.17, 128.26, 125.22, 124.72, 119.60, 118.35, 116.43, 105.88, 102.99, 100.04, 96.43, 72.75, 65.65, 52.08, 49.92, 34.16, 31.79, 30.61, 26.03, 24.16, 8.15.

### Reference Example 10 20(S)-7-(3-(4-morpholino)phenethyl))-10,11-methylenedioxy-campto thecin (compound 14)

(S,E)-7-ethyl-14-(3-(4-morpholinostyryl))-7-hydroxy-10,13-dihydro-11H-[1,3]diox olo[4,5-g]pyrano[3',4':6,7]indolizino[1,2-b]quinoline-8,11(7H)-dione was prepared and purified as described for the synthesis and purifying of compound 1 using 3-(4-morpholine)-benzaldehyde and then dissolved in EA at the present of Pb/C under hydrogen condition for 10 hrs. The mixture was filtered and the filtrate was concentrated, then purified by column chromatography (dichloromethane: acetone = 10:1) to afford compound 14; mp 163-165°C.

¹H NMR (500 MHz, DMSO) δ 7.61 (s, 1H), 7.47 (s, 1H), 7.20 (s, 1H), 7.07 (t, *J =* 7.8 Hz, 1H), 6.70 (d, *J =* 7.6 Hz, 1H), 6.64 (d, *J =* 7.5 Hz, 1H), 6.57 (s, 1H), 6.25 (s, 2H), 5.36 (s, 2H), 4.84 (d, *J =* 18.7 Hz, 1H), 4.73 (d, *J =* 18.7 Hz, 1H), 3.62 (s, 4H), 3.37 - 3.26 (m, 2H), 2.87 (s, 6H), 1.86 - 1.76 (m, 2H), 0.83 (t, *J =* 7.2 Hz, 3H).

¹³C NMR (125 MHz, DMSO) δ 172.95, 157.18, 151.43, 151.32, 150.56, 149.49, 149.39, 147.60, 146.69, 142.41, 141.91, 129.38, 128.36, 124.75, 120.21, 118.35, 116.11, 113.81, 105.89, 103.02, 100.04, 96.44, 72.77, 66.44, 65.62, 49.07, 35.56, 31.96, 30.61, 8.19.

### Reference Example 11 20(S)-7-(2-(4-morpholino)phenethyl))-10,11-methylenedioxy-campto thecin (compound 15)

(S,E)-7-ethyl-14-(2-(4-morpholinostyryl))-7-hydroxy-10,13-dihydro-11H-[1,3]dio xolo[4,5-g]pyrano[3',4':6,7]indolizino[1,2-b]quinoline-8,11(7H)-dione was prepared and purified as described for the synthesis and purifying of compound 1 using 2-(4-morpholine)-benzaldehyde and then dissolved in EA at the present of Pb/C under hydrogen condition for 10 hrs. The mixture was filtered and the filtrate was concentrated, then purified by column chromatography (dichloromethane: acetone = 10:1) to afford compound 15; mp >250°C.

¹H NMR (500 MHz, DMSO) δ 7.71 (s, 1H), 7.47 (s, 1H), 7.40 (d, *J =* 7.1 Hz, 1H), 7.19 - 7.14 (m, 2H), 7.07 (dd, *J =* 15.5, 7.5 Hz, 2H), 6.27 (s, 2H), 5.36 (s, 2H), 4.88 (d, *J* = 18.7 Hz, 1H), 4.76 (d, *J =* 18.6 Hz, 1H), 3.45 (d, *J =* 9.8 Hz, 4H), 3.31 - 3.20 (m, 2H), 3.00 (t, *J =* 7.4 Hz, 2H), 2.55 (dd, *J =* 34.3, 13.2 Hz, 4H), 1.81 (td, *J =* 14.5, 7.2 Hz, 2H), 0.83 (t, *J =* 7.3 Hz, 3H).

¹³C NMR (125 MHz, DMSO) δ 172.89, 157.14, 151.93, 151.26, 150.54, 149.51, 149.35, 147.50, 146.62, 142.41, 137.09, 131.07, 128.00, 127.89, 125.10, 124.97, 121.01, 118.40, 105.96, 103.07, 99.98, 96.39, 72.75, 66.77, 65.61, 53.52, 49.80, 32.39, 30.55, 30.44, 8.20.

### Reference Example 12 20(S)-7-(2-(pyrrolidin-1-yl)phenethyl)-10,11-methylenedioxy-campto thecin (compound 16)

(S,E)-7-ethyl-7-hydroxy-14-(3-(pyrrolidin-1-yl)styryl)-10,13-dihydro-11H-[1,3]di oxolo[4,5-g]pyrano[3',4':6,7]indolizino[1,2-b]quinoline-8,11(7H)-dione was prepared and purified as described for the synthesis and purifying of compound 1 using 2-(1-pyrrolidin)-benzaldehyde and then dissolved in EA at the present of Pb/C under hydrogen condition for 10 hrs. The mixture was filtered and the filtrate was concentrated, then purified by column chromatography (dichloromethane: acetone = 10:1) to afford compound 16; mp 139-140°C.

¹H NMR (500 MHz, DMSO) δ 7.66 (s, 1H), 7.47 (s, 1H), 7.18 (d, *J =* 7.5 Hz, 2H), 7.08 (d, *J =* 7.4 Hz, 1H), 6.99 (d, *J =* 8.0 Hz, 1H), 6.86 (t, *J =* 7.2 Hz, 1H), 6.26 (s, 2H), 5.37 (s, 2H), 4.95 (d, *J =* 18.7 Hz, 1H), 4.87 (d, *J =* 18.7 Hz, 1H), 3.26 (d, *J =* 10.4 Hz, 2H), 3.00 (t, *J =* 7.4 Hz, 2H), 2.96 - 2.87 (m, 4H), 1.86 - 1.75 (m, 6H), 0.83 (t, *J* = 7.3 Hz, 3H).

¹³C NMR (125 MHz, DMSO) δ 172.88, 157.19, 151.20, 150.44, 149.58, 149.28, 147.56, 147.46, 146.72, 142.52, 134.59, 131.16, 128.35, 127.61, 125.03, 122.68, 118.97, 112.07, 105.90, 103.01, 99.99, 96.35, 72.75, 65.59, 52.49, 34.53, 32.71, 30.56, 25.24, 8.18.

### Reference Example 13 20(S)-7-(4-(piperidin-1-yl)phenethyl)-10,11-methylenedioxy-camptot hecin (compound 17)

(S,E)-7-ethyl-7-hydroxy-14-(4-(piperidin-1-yl)styryl)-10,13-dihydro-11H-[1,3]dio xolo[4,5-g]pyrano[3',4':6,7]indolizino[1,2-b]quinoline-8,11(7H)-dione was prepared and purified as described for the synthesis and purifying of compound 1 using 4-(1-piperidin)-benzaldehyde and then dissolved in EA at the present of Pb/C under hydrogen condition for 10 hrs. The mixture was filtered and the filtrate was concentrated, then purified by column chromatography (dichloromethane: acetone = 10:1) to afford compound 17; mp >250°C.

¹H NMR (500 MHz, DMSO) δ 7.57 (s, 1H), 7.45 (s, 1H), 7.17 (s, 1H), 6.84 (s, 2H), 6.70 (s, 2H), 6.24 (s, 2H), 5.32 (t, *J =* 10.8 Hz, 2H), 4.64 (q, *J =* 18.8 Hz, 2H), 3.24 (d, *J* = 7.2 Hz, 2H), 2.97 (s, 4H), 2.81 (d, *J =* 4.8 Hz, 2H), 1.80 (dt, *J =* 23.4, 7.0 Hz, 2H), 1.45 (s, 6H), 0.83 (t, *J =* 7.3 Hz, 3H).

¹³C NMR (125 MHz, DMSO) δ 172.96, 157.11, 151.28, 150.46, 149.38, 147.53, 146.65, 142.33, 129.62, 128.44, 124.66, 118.28, 105.90, 103.02, 99.92, 96.40, 72.76, 65.64, 56.39, 49.83, 34.42, 32.39, 30.60, 25.12, 18.86, 8.17.

### Reference Example 14 20(S)-7-(3,5-difluoro-4-(4-morpholino)phenethyl)-10,11-methylenedi oxy-camptothecin (compound 18)

(S,E)-14-(3,5-difluoro-4-morpholinostyryl)-7-ethyl-7-hydroxy-10,13-dihydro-11 H-[1,3]dioxolo[4,5-g]pyrano[3',4':6,7]indolizino[1,2-b]quinoline-8,11(7H)-dione was prepared and purified as described for the synthesis and purifying of compound 1 using 3,5-difluoro-4(4-morpholin)-benzaldehyde and then dissolved in EA at the present of Pb/C under hydrogen condition for 10 hrs. The mixture was filtered and the filtrate was concentrated, then purified by column chromatography (dichloromethane: acetone = 10:1) to afford compound 18; mp 246-247°C.

¹H NMR (500 MHz, DMSO) δ 7.62 (s, 1H), 7.47 (s, 1H), 7.21 (s, 1H), 7.03 - 6.94 (m, 2H), 6.24 (s, 2H), 5.36 (d, *J =* 16.8 Hz, 2H), 5.10 (t, *J =* 11.5 Hz, 2H), 3.63 (s, 4H), 3.31 (s, 2H), 2.98 (s, 4H), 2.85 (s, 2H), 1.82 (d, *J =* 10.8 Hz, 2H), 0.83 (t, *J =* 7.1 Hz, 3H).

¹³C NMR (125 MHz, DMSO) δ 172.98, 157.24, 154.12, 151.31, 150.56, 149.53, 149.40, 147.54, 146.67, 142.16, 138.44, 136.18, 128.15, 125.36, 124.72, 119.24, 118.35, 116.75, 116.59, 105.81, 103.02, 99.99, 96.56, 72.77, 66.54, 65.65, 51.07, 49.91, 34.12, 31.77, 30.64, 8.16.

### Reference Example 15 20(S)-7-(3,5-difluoro-4-(pyrrolidin-1-yl)phenethyl)-10,11-methylened ioxy-camptothecin (compound 19)

(S,E)-14-(3,5-difluoro-4-(pyrrolidin-1-yl)styryl)-7-ethyl-7-hydroxy-10,13-dihydro -11H-[1,3]dioxolo[4,5-g]pyrano[3',4':6,7]indolizino[1,2-b]quinoline-8,11(7H)-dione was prepared and purified as described for the synthesis and purifying of compound 1 using 3,5-difluoro-4-(1-pyrrolidin)-benzaldehyde and then dissolved in EA at the present of Pb/C under hydrogen condition for 10 hrs. The mixture was filtered and the filtrate was concentrated, then purified by column chromatography (dichloromethane: acetone = 10:1) to afford compound 19; mp>250°C.

¹H NMR (500 MHz, DMSO) δ 7.57 (s, 1H), 7.44 (s, 1H), 7.21 (s, 1H), 6.90 - 6.84 (m, 2H), 6.24 (d, *J =* 1.6 Hz, 2H), 5.37 (t, *J =* 11.8 Hz, 2H), 5.01 (d, *J =* 1.9 Hz, 2H), 3.27 (d, *J =* 17.6 Hz, 6H), 2.82 - 2.76 (m, 2H), 1.89 - 1.77 (m, 6H), 0.86 (t, *J =* 7.3 Hz, 3H).

¹³C NMR (125 MHz, DMSO) δ 172.96, 157.24, 156.21, 154.35, 151.28, 150.54, 149.60, 149.38, 147.53, 146.68, 141.96, 133.30, 128.01, 124.67, 124.47, 118.38, 113.03, 112.84, 105.79, 103.02, 99.99, 96.49, 72.76, 65.67, 51.29, 49.92, 33.83, 31.45, 30.66, 25.45, 8.17.

### Reference Example 16 20(S)-7-(4-(4-thiomorpholino)phenethyl)-10,11-methylenedioxy-cam ptothecin (compound 20)

(S,E)-7-ethyl-7-hydroxy-14-(4-thiomorpholinostyryl)-10,13-dihydro-11H-[1,3] dioxolo[4,5-g]pyrano[3',4':6,7]indolizino[1,2-b]quinoline-8,11(7H)-dione was prepared and purified as described for the synthesis and purifying of compound 1 using 4-thiomorpholin-benzaldehyde and then dissolved in EA at the present of Pb/C under hydrogen condition for 10 hrs. The mixture was filtered and the filtrate was concentrated, then purified by column chromatography (dichloromethane: acetone = 10:1) to afford compound 20; mp154-156°C.

¹H NMR (600 MHz, DMSO-D6) δ 7.63 (s, 1H), 7.49 (s, 1H), 7.20 (s, 1H), 6.81 (d, *J = 8.6* Hz, 2H), 6.67 (d, *J =* 8.6 Hz, 2H), 6.28 (d, *J =* 2.9 Hz, 2H), 5.42 - 5.32 (m, 2H), 4.58 (q, *J =* 18.2 Hz, 2H), 3.44 (d, *J =* 4.9 Hz, 4H), 3.30 (d, *J =* 8.0 Hz, 2H), 2.90-2.84 (m, 2H), 2.46 (d, *J =* 2.8 Hz, 4H), 1.87 - 1.76 (m, 2H), 0.85 (t, *J =* 7.4 Hz, 3H).

¹³C NMR (125 MHz, DMSO) δ 172.95, 157.07, 151.28, 150.32, 149.38, 149.27, 148.52, 147.53, 146.56, 142.26, 130.65, 130.02, 128.72, 124.71, 118.41, 115.97, 105.92, 103.03, 99.97, 99.17, 96.39, 72.72, 65.75, 51.24, 49.80, 34.40, 32.43, 30.65, 24.72, 8.19.
The synthesis scheme of compounds **21-42** was as follows:

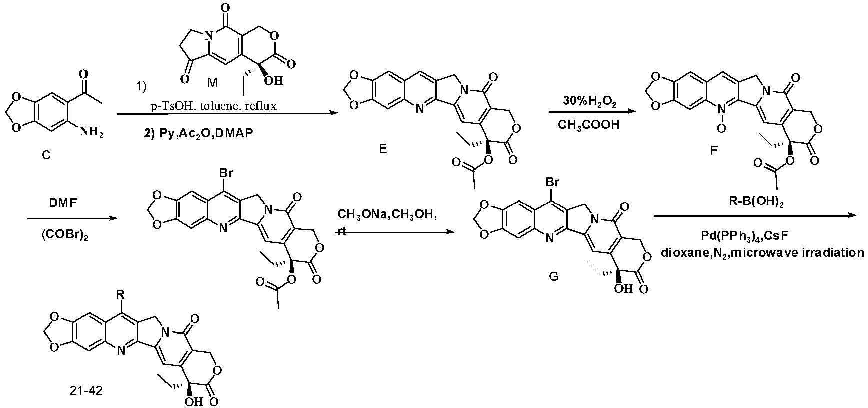

### Example 17 20(S)-7-(pyridin-4-yl)-10,11-methylenedioxy-camptothecin (compou nd 21)

1) The intermediate C obtained in step (1) of example 1 was directly condensed and cyclized according to the method in the step (2) of example 1, and then t he intermediate E was obtained by protecting the hydroxyl group with acetic an hydride in the presence of pyridine and DMAP.
2) 30% hydrogen peroxide solution (3.2mL, 28mmol) was added to the mixture of 20 (S)-O-acetyl-10,11-methylenedioxy-camptothecin (0.24g, 0.55mmol) and acetic acid (20mL). The mixture reacted at 75°C for 3 hrs. The mixture was cooled and the solvent was removed under reduced pressure to afford **F,** yellow solid, yield: 210 mg (85%).
3) Oxalyl bromide (0.1mL, 1.1mmol) was added to the mixture of the compound **F** we got (0.21g, 0.47mmol) in DMF (20mL) at 0°C. The mixture reacted at 15°C for 3hrs and then poured into ice water (50ml), extracted with dichloromethane for 3 times. The organic layer was combined, dried by anhydrous MgSO₄ and concentrated. Then it was purified by column chromatography (dichloromethane: methanol=100:1) to afford **G,** yellow solid, yield: 0.16 g (75%). mp >250°C.

¹H NMR (500 MHz, CF₃COOD) δ 8.14 (s, 1H), 7.89 (s, 1H), 7.71 (s, 1H), 6.47 (s, 2H), 5.93 (d, *J =* 17.2 Hz, 1H), 5.72 (s, 2H), 5.59 (d, *J =* 17.1 Hz, 1H), 2.16 (q, *J =* 7.4 Hz, 2H), 1.15 (t, *J =* 7.4 Hz, 3H).

¹³C NMR (125 MHz, CF₃COOD) δ 176.01, 158.41, 153.96, 151.23, 139.54, 139.37, 138.51, 138.21, 131.56, 128.70, 125.52, 122.45, 109.98, 105.38, 102.83, 97.44, 73.68, 66.17, 53.26, 31.09, 5.76.
4) In a 25 mL two round bottom flask , 20(*S*)-O-acetyl-7-bromide-10,11-methylenedioxy-camptothecin **G** (37.6 mg, 0.08 mmol), pyridyl-4-boric acid (20 mg, 0.16 mmol), cesium fluoride (25 mg, 0.16 mmol), Pd(PPh₃)₄ (9.2 mg, 0.008 mmol) and dioxane (5mL), ethnol (1.9 mL) and water (3.2 mL) were added in sequence. The mixture reacted under microwave and nitrogen conditionat 105°C for 6 hrs. The mixture was cooled and the solvent was removed under reduced pressure. Then it was purified by column chromatography (dichloromethane:methnol=10:1) to afford **21,** yellow solid, yield: 19 mg (50.3%). mp >250°C.

¹H NMR (500 MHz, CF₃COOD) δ 9.32 (s, 2H), 8.48 (dd, *J =* 13.7, 5.3 Hz, 2H), 8.22 (s, 1H), 7.84 (d, *J =* 11.1 Hz, 1H), 7.13 (d, *J =* 6.5 Hz, 1H), 6.45 (s, 2H), 5.87 (d, *J* = 17.5 Hz, 1H), 5.56 (t, *J =* 14.9 Hz, 3H), 2.19 - 2.10 (m, 2H), 1.13 (dd, *J =* 16.1, 8.7 Hz, 3H).

¹³C NMR (125 MHz, CF₃COOD) δ 175.93, 162.76, 160.89, 158.41, 154.08, 151.76, 151.25, 144.14, 143.34, 141.25, 139.92, 138.84, 128.06, 126.43, 124.82, 114.19, 105.68, 104.70, 100.41, 97.95, 73.62, 66.04, 51.11, 31.06, 5.67.

### Example 18 20(S)-7-(pyridin-3-yl)-10,11-methylenedioxy-camptothecin (compou nd 22)

The synthesis and purification of Compound **22** was described as compound 21, only pyridyl-4-boric acid was substituted by pyridyl-3-boric acid. Yellow solid 23 mg (62%), mp 245-246°C.

¹H NMR (500 MHz, CF₃COOD) δ 9.39 (d, *J =* 23.3 Hz, 1H), 9.26 (d, *J =* 5.8 Hz, 1H), 8.98 (dd, *J* = 14.9, 8.0 Hz, 1H), 8.51 (t, *J* = 7.1 Hz, 1H), 8.18 (s, 1H), 7.80 (d,*J =* 4.7 Hz, 1H), 7.13 (d, *J =* 2.4 Hz, 1H), 6.42 (d, *J =* 5.7 Hz, 2H), 5.85 (d, *J =* 17.3 Hz, 1H), 5.58 - 5.49 (m, 3H), 2.12 (d, *J =* 7.6 Hz, 2H), 1.12 (t, *J =* 7.3 Hz, 3H).

¹³C NMR (125 MHz, CF₃COOD) δ 175.96, 158.35, 157.70, 154.05, 151.22, 147.63, 143.39, 142.60, 141.88, 141.12, 139.87, 138.95, 133.06, 128.98, 127.58, 122.52, 109.99, 105.65, 104.68, 100.55, 97.86, 73.66, 66.09, 31.07, 18.44, 5.67.

### Example 19 20(S)-7-(furan-3-yl)-10,11-methylenedioxy-camptothecin (compoun d 23)

The synthesis and purification of Compound **23** was described as compound 21, only pyridyl-4-boric acid was substituted by 3-furan boric acid. Yellow power 23 mg (62%), mp 246-247°C.

¹H NMR (500 MHz, CF₃COOD) δ 8.14 (s, 1H), 8.07 (s, 1H), 7.85 (s, 1H), 7.77 (s, 1H), 7.69 (d, *J =* 12.2 Hz, 1H), 6.88 (s, 1H), 6.42 (d, *J =* 24.8 Hz, 2H), 5.89 (d, *J =* 16.6 Hz, 1H), 5.61 (dd, *J =* 50.1, 25.2 Hz, 3H), 2.13 (s, 2H), 1.13 (s, 3H).

¹³C NMR (125 MHz, CF₃COOD) δ 176.07, 157.97, 157.61, 152.85, 151.26, 145.88, 144.05, 140.68, 140.07, 139.79, 139.13, 128.06, 127.08, 109.47, 105.36, 104.93, 103.85, 102.25, 97.47, 97.24, 73.74, 66.15, 52.07, 31.01, 5.71.

### Example 20 20(S)-7-(thiophene-3-yl)-10,11-methylenedioxy-camptothecin (comp ound 24)

The synthesis and purification of Compound **24** was described as compoun d 21, only pyridyl-4-boric acid was substituted by 3-thiophene boric acid. Yello w power, yield: 23 mg (62%), mp 237-238°C.

¹H NMR (500 MHz, CF₃COOD) δ 8.15 (s, 1H), 7.91 (s, 1H), 7.84 (s, 1H), 7.69 (s, 1H), 7.63 (s, 1H), 7.45 (d, *J =* 4.6 Hz, 1H), 6.40 (s, 2H), 5.90 (d, *J =* 17.1 Hz, 1H), 5.56 (d, *J =* 15.6 Hz, 2H), 2.15 (q, *J =* 7.2 Hz, 2H), 1.14 (t, *J =* 7.3 Hz, 3H).

¹³C NMR (125 MHz, CF₃COOD) δ 176.08, 157.58, 152.77, 151.24, 149.15, 140.04, 139.83, 139.33, 131.51, 128.93, 128.64, 128.31, 127.41, 126.44, 121.64, 109.99, 104.88, 103.77, 102.55, 97.08, 73.74, 66.14, 51.89, 30.98, 5.68.

### Example 21 20(S)-7-(1-methyl-1H-pyrazol-4-yl)-10,11-methylenedioxy-camptoth ecin (compound 25)

The synthesis and purification of Compound **25** was described as compound 21, only pyridyl-4-boric acid was substituted by 4-(1-methyl-1H-pyrazol)-boric. Yell ow power, mp 237-238°C.

¹H NMR (500 MHz, CF₃COOD) δ 8.76 (s, 1H), 8.70 (s, 1H), 8.17 (s, 1H), 7.75 (s, 1H), 7.59 (s, 1H), 6.45 (s, 2H), 5.91 (d, *J =* 17.2 Hz, 1H), 5.72 (d, *J =* 3.1 Hz, 2H), 5.57 (d, *J =* 17.1 Hz, 1H), 4.50 (s, 3H), 2.16 (q, *J =* 7.3 Hz, 2H), 1.15 (t, *J =* 7.3 Hz, 3H).

¹³C NMR (125 MHz, CF₃COOD) δ 176.02, 158.09, 157.73, 153.71, 151.21, 140.58, 139.68, 139.52, 139.23, 136.11, 135.92, 129.22, 127.27, 122.20, 109.99, 105.41, 104.17, 101.01, 97.61, 73.68, 66.09, 51.85, 38.46, 31.03, 5.67.

### Example 22 20(S)-7-(thiophene-2-yl)-10,11-methylenedioxy-camptothecin (compound 26)

The synthesis and purification of Compound **26** was described as compoun d 21, only pyridyl-4-boric acid was substituted by 2-thiophene boric acid. Yello w power, mp 240-241°C.

¹H NMR (500 MHz, CF₃COOD) δ 8.15 (s, 1H), 7.99 (d, *J =* 5.0 Hz, 1H), 7.87 (s, 1H), 7.69 (d, *J =* 4.2 Hz, 2H), 7.51 (t, *J =* 4.1 Hz, 1H), 6.42 (s, 2H), 5.91 (d, *J =* 17.0 Hz, 1H), 5.68 (s, 2H), 5.57 (d, *J =* 17.0 Hz, 1H), 2.16 (q, *J =* 7.3 Hz, 2H), 1.15 (t, *J* = 7.3 Hz, 3H).

¹³C NMR (125 MHz, CF₃COOD) δ 176.08, 157.57, 153.02, 151.24, 146.96, 140.00, 139.77, 139.42, 132.81, 132.01, 131.02, 128.78, 128.19, 127.21, 121.69, 109.99, 104.97, 103.75, 102.77, 97.17, 73.75, 66.15, 52.35, 30.99, 5.68.

### Example 23 20(S)-7-(furan-2-yl)-10,11-methylenedioxy-camptothecin (compound 27)

The synthesis and purification of Compound **27** was described as compound 21, only pyridyl-4-boric acid was substituted by 2-furan boric acid. Yellow power, mp >250°C.

¹H NMR (500 MHz, CF₃COOD) δ 8.19 (s, 1H), 8.12 (d, *J =* 15.7 Hz, 2H), 7.71-7.67 (m, 1H), 7.64 (s, 1H), 7.00 (s, 1H), 6.44 (d, *J =* 16.8 Hz, 2H), 5.93 (d, *J =* 23.4 Hz, 3H), 5.59 (d, *J =* 16.9 Hz, 1H), 2.16 (d, *J =* 7.2 Hz, 2H), 1.15 (t, *J =* 7.1 Hz, 3H).

¹³C NMR (125 MHz, CF₃COOD) δ 176.08, 157.58, 152.77, 151.24, 149.15, 140.04, 139.83, 139.33, 131.51, 128.93, 128.64, 128.31, 127.41, 126.44, 121.64, 110.80, 104.88, 103.77, 102.55, 97.08, 73.74, 66.14, 51.89, 30.98, 5.68.
The synthesis scheme of compounds **43-46** was as follows:

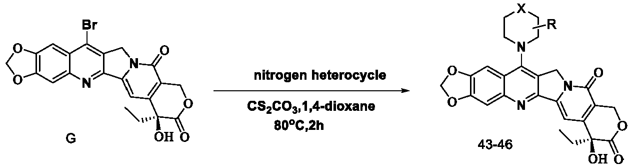

### Reference Example 24 20(S)-7-morpholino-10,11-methylenedioxy-camptothecin (compoun d 43)

In a 25 mL two round bottom flask , 20(S)-O-acetyl-7-bromide-10,11-methylenedioxy-camptothecin **G** (37.6 mg, 0.08 mmol), morpholin (1 mL), cesium fluoride (52 mg, 0.16 mmol) and dioxane (15mL) were added in sequence. The mixture reacted under nitrogen condition at 85°C overnight. The mixture was cooled and the solvent was removed under reduced pressure. Then it was purified by column chromatography (dichloromethane:methnol=10:1) to afford compound **43,** brown solid, yield:19 mg (50.3%). mp >250°C.

¹H NMR (500 MHz, CF₃COOD) δ 8.14 (s, 1H), 7.89 (s, 1H), 7.71 (s, 1H), 6.47 (s, 2H), 5.93 (d, *J =* 17.2 Hz, 1H), 5.72 (s, 2H), 5.59 (d, *J =* 17.1 Hz, 1H), 3.37 - 3.26 (m, 2H), 2.87 (s, 6H), 2.16 (q, *J =* 7.4 Hz, 2H), 1.15 (t, *J =* 7.4 Hz, 3H).

¹³C NMR (125 MHz, CF₃COOD) δ 176.01, 158.41, 153.96, 151.23, 139.54, 139.37, 138.51, 138.21, 131.56, 128.70, 125.52, 122.45, 109.98, 105.38, 102.83, 97.44, 73.68, 66.17, 53.26, 35.56, 31.96, 31.09, 30.61, 5.76.
The synthesis scheme of compounds **47-50** was as follows:

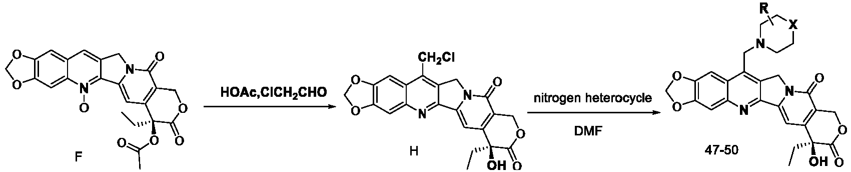

### Reference Example 25 20(S)-7-(morpholinomethyl)-10,11-methylenedioxy-camptothecin (Compound 47)

### 1)20 (S)-7-methyl chloride-10,11-methylenedioxy-camptothecin (H)

In a 25 mL two round bottom flask, intermediates **F** (37.6 mg, 0.08 mmol), ice acetic acid (1 mL) were mixed and then added 2-chloroacetaldehyde (0.5 mL). The mixture reacted at room temperature overnight. The mixture was cooled and the solvent was removed under reduced pressure. Then it was purified by column chromatography (dichloromethane:methnol=10:1) to afford **H,** yellow solid, yield:21 mg (60.3%). mp >250°C.

¹H NMR (500 MHz, DMSO) δ 8.45 (s, 1H), 7.49 (s, 2H), 7.24 (s, 1H), 6.47 (s, 1H), 6.27 (s, 2H), 5.39 (d, *J =* 16.5 Hz, 2H), 5.20 (s, 2H), 4.52 (s, 2H),1.90 - 1.79 (m, 2H), 0.91 - 0.81 (m, 3H).

¹³C NMR (125 MHz, DMSO) δ 172.98, 157.28, 151.78, 150.50, 150.41, 14 9.09, 147.03, 146.37, 130.60, 128.88, 126.06, 118.54, 105.29, 103.56, 103.03, 96. 34, 72.85, 65.69, 62.21, 50.63, 30.68, 8.23.

### 2)20(S)-7-(morpholinomethyl)-10,11-methylenedioxy-camptothecin (Compound 47)

The mixture of intermediates **H** (26.4 mg, 0.08 mmol), morpholin (3 mL) and DMF (10 mL) reacted at room temperature overnight. The mixture was cooled and the solvent was removed under reduced pressure. Then it was purified by column chromatography (dichloromethane:methnol=10:1) to afford **47,** yellow solid, yield: 13 mg (33.3%). mp >250°C.

¹H NMR (500 MHz, DMSO) δ 8.45 (s, 1H), 7.49 (s, 2H), 7.24 (s, 1H), 6.47 (s, 1H), 6.27 (s, 2H), 5.39 (d, *J =* 16.5 Hz, 2H), 5.20 (s, 2H), 4.52 (s, 2H), 3.37 - 3.26 (m, 2H), 2.87 (s, 6H), 1.90 - 1.79 (m, 2H), 0.91 - 0.81 (m, 3H).

¹³C NMR (125 MHz, DMSO) δ 172.98, 157.28, 151.78, 150.50, 150.41, 149.09, 147.03, 146.37, 130.60, 128.88, 126.06, 118.54, 105.29, 103.56, 103.03, 96.34, 72.85, 65.69, 62.21, 50.63, 35.56, 31.96, 31.09, 30.68, 30.61, 8.23.
The synthesis scheme of compounds 51-54 was as follows:

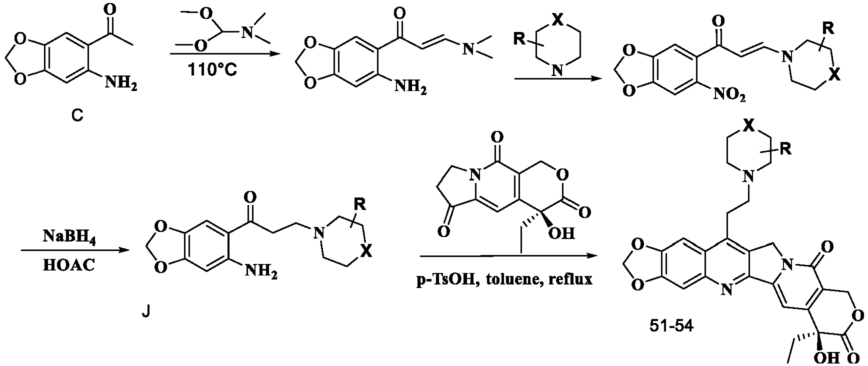

### Reference Example 26 20(S)-7-(2-morpholinoethyl)-10,11-methylenedioxy-camptothecin (compound 51)

1) (E)-1-(6-aminobenzo[d][1,3]dioxol-5-yl)-3-(dimethylamino)prop-2-en-1-one Intermediates C (7.6 g, 37.3 mmol) dissolved in N,N-dimethylformamide dimethyl acetal and reacted at 110°C for 2 hrs. The mixture was cooled and the solvent n-hexane was added to get yellow solid 9.4 g (95%). The solid (9.4 g, 35.6 mmol) and morpholin (3.6 mL, 36 mmol) are mixed in 60 mL dioxane under refluxing for 6 hrs. Then cooled down and removed the solvent under reduced pressure. Then it was purified by column chromatography (petroleum ehter:ethyl acetate=1:2) to afford yellow solid 8.7 g (80%). Dissolved the solid in 20 mL ice acetic acid, then added the sodium borohydride (493.1 mg, 13.1 mmol) at 0°C and stirred for 3 hrs. Removed the solvent under reduced pressure. Then it was purified by column chromatography (petroleum ehter:ethyl acetate=1:2) to afford yellow solid 5.6 g (70%).
   ¹H NMR (500 MHz, DMSO) δ 7.34 (s, 2H), 7.24 (s, 1H), 6.29 (s, 1H), 5.92 (s, 2H), 3.57 - 3.50 (m, 2H), 2.94 (t, *J =* 7.4 Hz, 2H), 2.57 (t, *J =* 7.3 Hz, 2H), 2.37 (s, 4H).
2)20(S)-7-(2-morpholinoethyl)-10,11-methylenedioxy-camptothecin (compound 51) (4S)-4-Ethyl-7,8-dihydro-4-hydroxy-1H-pyrano[3,4-f]indolizine-3,6,10(4H)-trione (200 mg, 0.76 mmol) was dissolved in anhydrous toluene (70 mL), 6'-amino-3',4'-methylenedioxy-morpholinylethyl benzophenone (0.38 g, 1.37 mmol) and para-toluenesulfonic (26.2 mg, 0.15 mmol) were added. The mixture reacted at 110°C under nitron for 24 hrs. The mixture was cooled and the solvent was removed under reduced pressure. The compounds were purified by column chromatography (dichloromethane: methanol=97:3) to afford **51,** light yellow solid, yield: 150 mg (40%). mp >250°C.

¹H NMR (CF₃COOD, 600 MHz) δ: 7.26 (s, 1 H), 6.98 (s, 1 H), 6.74 (s, 1 H), 5.9 (s, 2 H), 4.76 (d, 1 H), 4.74 (d, 1 H), 4.22 (s, 2 H), 3.67 (m, 4 H), 2.69 (d, 2 H), 2.65 (d, 2 H), 2.37 (d, 4 H), 1.87 (m, 2 H), 0.96 (t, 3 H).

¹³C NMR (CF₃COOD, 150 MHz) δ: 178.6, 159.4, 157.7, 156.9, 151.7, 149.4, 145.8, 141.4, 139.2, 126.2, 123.5, 120.9, 108.3, 102.2, 101.5, 101.4, 73.0, 66.8, 58.1, 55.5, 53.7,45.6, 30.2, 28.5,28.4, 26.2, 5.6.

### Reference Example 27 20(S)-7-(2-thiomorpholinoethyl)-10,11-methylenedioxy-camptotheci n (compound 54)

Compound 54 was prepared and purified as described for the synthesis and purifying of **51.** Thiomorpholine is used instead of morpholinr in step (1) of example 24. Other raw materials and reagents used are the same. Using
(4S)-4-Ethyl-7,8-dihydro-4-hydroxy-1H-pyrano[3,4-f]indolizine-3,6,10(4 H)-trione and 6'-amino-3',4'-methylenedioxy-thiomorpholinylethyl benzophenone, we got compound **54,** light yellow solid, yield: 170 mg (45%). mp >250°C.

¹H NMR (CF₃COOD, 600 MHz) δ: 7.26 (s, 1 H), 6.98 (s, 1 H), 6.74 (s, 1 H), 5.9 (s, 2 H), 4.76 (d, 1 H), 4.74 (d, 1 H), 4.22 (s, 2 H), 2.73 (m, 4 H), 2.69 (d, 2 H), 2.65 (d, 2 H), 2.54 (d, 4 H), 1.87 (m, 2 H), 0.96 (t, 3 H).

¹³C NMR (CF₃COOD, 150 MHz) δ: 172.6, 159.4, 157.7, 156.9, 151.7, 149.4, 145.8, 141.4, 139.2, 126.2, 123.5, 120.9, 108.3, 102.2, 101.5, 101.4, 73.0, 65.1, 58.3, 58.1, 55.5, 45.6, 30.2, 28.5, 28.4, 26.2, 5.6.

### Reference Example 28 20(S)-7-(2-(piperidin-1-yl)ethyl)-10,11-methylenedioxy-camptotheci n (compound 52) and 20(S)-7-(2-(2-methylpiperidin-1-yl)ethyl)-10,11-methylene dioxy-camptothecin (compound 53)

Compound **52** was prepared and purified as described for the synthesis and purifying of **51,** and piperdine is used to replace morpholine in step (1) of exa mple 24. Other raw materials and reagents used are the same. Compound **53** w as prepared and purified as described for the synthesis and purifying of **51, an d** 2-methyl-piperidine is used to replace morpholine in step (1) of example 24.
The synthesis scheme of introducing different thiazole substituent at 7 position of 10, 11-methylene-camptothecin derivatives are as follows:

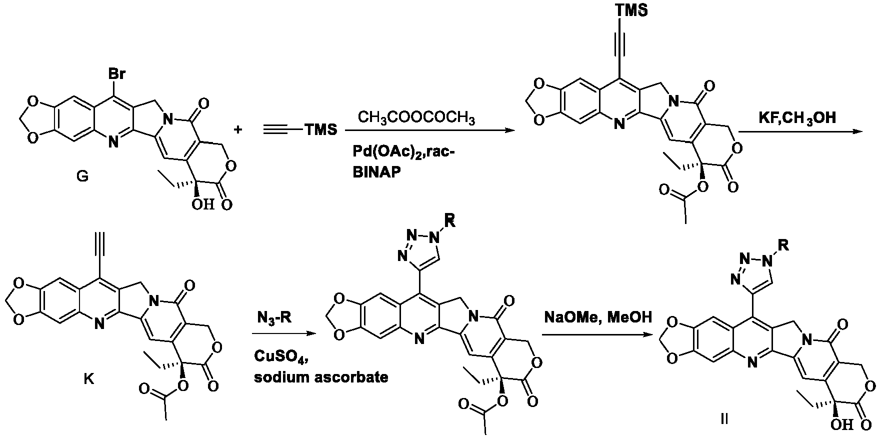

### Reference Example 29 20(S)-7-(1-butyl-1H-1,2,3-triazol-4-yl)-10,11-methylenedioxy-campto thecin (compound 55)

### 1) 20 (S)-O-acetyl-7-(2- trimethylsilylaceyene)-10,11- methylenedioxy-camptothecin

To a mixture of 20(*S*)-O-acetyl-7-chloro-10,11-methylenedioxy-camptothecin (0.47g, 1mmol), palladium acetate(45mg, 0.2mmol), rac-BINAP(0.25g, 0.4mmol) and potassium carbonate(0.28g, 2mmol), trimethylsilylaceyene(17mL, 0.8mmol) dissolved in toluene were added under nitrogen. The mixture reacted at 100°C for 9 hrs. The mixture was cooled and the solvent was removed under reduced pressure. The compounds were purified by column chromatography (dichloromethane: acetone=30:1) to afford 350 mg light yellow solid (65%). mp >250°C. The compounds we got above (0.35g, 0.66mmol), potassium fluoride (77mg, 1.32mmol) and anhydrous methanol (25mL), reacted at room temperature for 2hrs. The solvent was removed under reduced pressure and water was added. The mixture was extracted with chloroform for three times. The organic layer was combined, dried by anhydrous MgSO₄ and concentrated. The yellow oil we got was purified by column chromatography (dichloromethane: acetone=30:1) to afford **K,** yellow solid, yield:0.24 g (80%). mp >250°C.

¹H NMR (CF₃COOD, 600 MHz) δ: 7.29 (s, 1 H), 6.88 (s, 1 H), 6.74(s, 1 H), 5.90 (s, 2 H), 4.76 (d, 1 H), 4.74 (d, 1 H), 4.22 (s, 2 H), 3.06 (s, 1 H), 2.04 (d, 1 H), 1.96 (m, 2 H), 0.96 (t, 3 H).

¹³C NMR (CF₃COOD, 150 MHz) δ: 172.5, 170.3, 159.7, 157.3, 157.0, 150.7, 149.9, 145.5, 141.0, 130.6, 129.1, 126.8, 120.0, 108.1, 106.3, 101.6, 101.2, 76.0, 73.5, 69.7, 65.5, 43.8, 27.0, 21.1, 5.8.

### 2) 20 (S)-7-(1-butyl-1H-1,2,3- triazole-4-)-10,11- methylenedioxy-camptothecin.

20(S)-O-acetyl-7-acetenyl-10,11-methylenedioxy-camptothecin(0.24 g, 0.52 mmol), tertiary butanol (10mL) and water(10mL) were mixed. 1-butyl-azido(0.10 g, .04mmol), copper sulfate solvent(10 mmol/L, 0.005 mmol) and sodium ascorbate(0.02g, 0.1mmol) were added in sequence to the mixture. The mixture reacted at 60°C for 6 hrs. The mixture was poured to ice water and extracted with chloroform for three times. The organic layer was combined, dried by anhydrous MgSO₄ and concentrated. The compounds were purified by column chromatography (dichloromethane: acetone=30:1)to afford yellow solid, yield: 0.23g (80%). mp >250°C. Sodium methoxide (0.05g, 0.84mmol) was added to the mixture of 20 (*S*)-O-acetyl-7-(1-butyl-1H-1,2,3-triazole-4-)-10,11-methylenedioxy-camptothecin(0.2 3g, 0.41mmol) and methanol (20mL). The mixture reacted at room temperature for 6 hrs. pH of the solution was adjusted to 7.0. The solvent was removed under reduced pressure. The compounds were purified by column chromatography (dichloromethane: acetone=30:1) to afford **55,** yellow solid, yield:0.18 g (85%). mp >250°C.

¹H NMR (CF₃COOD, 600 MHz) δ: 7.6 (s, 1 H), 7.29 (s, 1 H), 6.88(s, 1 H), 6.74(s, 1 H), 5.90 (s, 2 H), 4.76 (d, 1 H), 4.74 (d, 1 H), 4.22 (s, 2 H), 3.73 (t, 2 H), 1.87 (m, 2 H), 1.77 (m, 2 H), 1.33 (m, 2 H), 0.98 (t, 2 H), 0.96 (t, 3 H).

¹³C NMR (CF₃COOD, 150 MHz) δ: 172.5, 159.7, 157.3, 157.0, 151.6, 149.9, 146.3, 145.5, 141.9, 141.0, 130.4, 124.0, 120.0, 119.1, 108.1, 106.3, 101.6, 101.2, 73.5, 65.8, 52.2, 44.6, 30.6, 30.3, 30.3, 20.3, 13.8, 5.8.

### Reference Example 30 Synthesis of intermediate M

The synthesis scheme of M was as follows:

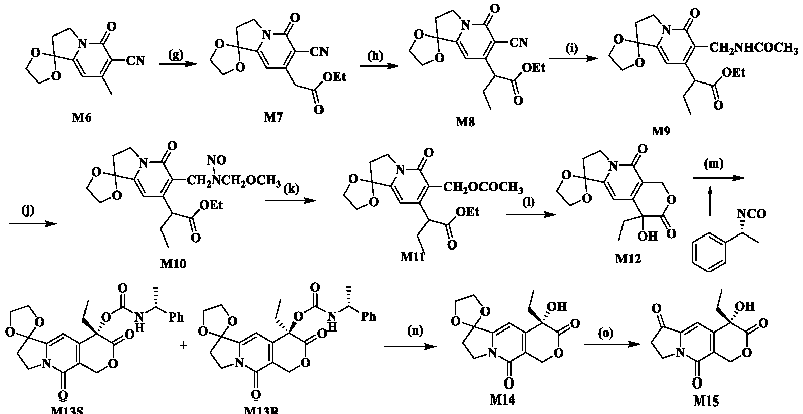

Reagents and conditions: (g) CO(OEt)₂, t-BuOK, THF; (h) C₂H₅Br, K₂CO₃, CH₃CN; (i) H₂, Raney Ni, HOAC, Ac₂O; (j) NaNO₂, 0°C; (k) CCl₄, reflux; (l) 1) O₂, Raney Ni , CuCl, 2) H₂SO₄; (m) CuCl, CH₂Cl₂; (n) KOH, CH₃OH; (o) CF₃COOH, r.t.

Generally, Compound **M6** reacted with diethyl carbonate at the present of potassium tert-butoxide to yield intermediate M7; M9 was got by reacting with bromoethane and then hydrogenation by Ni/H₂; M9 reacted with sodium nitrite to get M10 and then through rearrangement in CCl₄ and oxidization to obtain M12 which was a chiral compound. Chiral isocyanates was used to react with M12 catalyzed by copper chloride and we can get two non-corresponding isomers through column chromatography; M13S was hydrolyzed by base and deprotected by TFA to obtain M15.

### 1) Synthesis of ethyl 2-(6-cyano-5-oxo-2,3-dihydro-5H-spiro[indolizine-1,2'-[1,3]dio xolan]-7-yl)acetate (M7)

**M6** (20 g, 86 mmol) was dissolved in 120 mL anhydrous tetrahydrofuran and then added the potassium tert-butyl alcohol (43.43 g, 387 mmol) and diethyl carbonate (40.6 g, 344 mmol). The mixture was refluxing for 5 hrs. Then cooled down to room temperature and quenched the reaction by adding 5 mL ice acetic acid to get brown solid. The mixture were purified by column chromatography (dichloromethane: ethyl acetate=6:1)to **M7,** afford yellow solid, yield: 23.65 g (90%). mp 172-173°C.

### 2) Synthesis of ethyl 2-(6-cyano-5-oxo-2,3-dihydro-5H-spiro[indolizine-1,2'-[1,3]dio xolan]-7-yl)butanoate (M8)

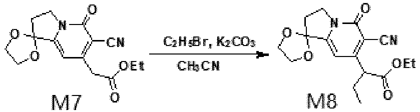

Mixed the compound **M7** (20 g, 65.64 mmol), potassium carbonate (24 g, 172 mmol) and bromine ethane (17.58 g, 162 mmol) in 300 mL acetonitrile and stired at 85 °C for 3 hrs. Then cooled down to room temperature and filter the inorganic salt and washed the residue using acetonitrile for three times. The filtrate was concentrated under reduced pressure to get **M8,** yellow solid 21.82 g (95%).

### 3) Synthesis of ethyl 2-(6-(acetamidomethyl)-5-oxo-2,3-dihydro-5H-spiro[indolizine -1,2'-[1,3]dioxolan]-7-yl)butanoate (M11)

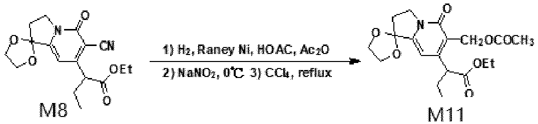

M8 (12 g, 36.1 mmol) was dissolved in the mixture of acetic anhydride (150 mL) and ice acetic acid (50 mL), then Rainie Ni (6 g) was added under hydrogen at 45°C for 6 hrs. Filter the catalyst and washed the residue using acetic acid for three times. The filtrate was concentrated under reduced pressure to get yellow oil liquid. Added the mixture made of acetic anhydride (150 mL), ice acetic acid (50 mL) and sodium nitrite (13 g, 188 mmol) into the oil liquid and stired for 2 hrs. Filter the inorganic salt and washed the residue using acetic acid for three times. The filtrate was concentrated under reduced pressure to get yellow oil liquid. Refluxing for 12 hrs in 200 mL carbon tetrachloride and washed the solvent with water, saturated salt water for three times. Combined the organic layer and concentrated under reduced pressure to get **M11,** yellow oil liquid, yield: 16.2 g (80%).

### 4) Synthesis of 4-ethyl-4-hydroxy-1,4,7,8-tetrahydro-3H,10H-spiro[pyrano[3,4-f]indo lizine-6,2'-[1,3]dioxolane]-3,10-dione (M12)

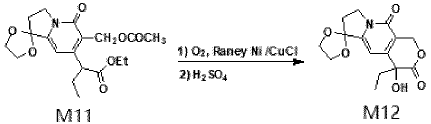

The mixture of Raney Ni (3 g, 51.1 mmol), cuprous chloride (0.2 g, 2 mmol) and anhydrous potassium carbonate (8 g, 57.9 mmol) were dissolved in 300 mL methanol under N₂ for 1 hr. Then added the **M11** (21 g, 53 mmol) under O₂ for 6 hrs. Filter the catalyst and concentrated under reduced pressure to get yellow oil liquid. 200 mL water was added in the product and adjusted the pH to 2-3 using sulphuric acid. The mixture was extracted by dichloromethane for three times and combined the organic layer and concentrated under reduced pressure to get **M12,** white solid, yield: 8 g (49%), mp 177-179°C.

¹H NMR (CDCl₃, 600 MHz) δ: 6.57 (s, 1 H, Ar-H), 5.16-5.63 (m, 2 H, ArCH2O), 4.16 (m, 6 H, OCH2CH2O, NCH2), 3.69 (s, 1 H, OH), 2.42 (t, 2 H, NCH2CH2), 1.81 (m, 2 H, CH2CH3), 0.98 (t, 3 H, CH3).

### 5) Synthesis of (R)-4-ethyl-3,10-dioxo-4,7,8,10-tetrahydro-1H,3H-spiro[pyrano[3,4-f] indolizine-6,2'-[1,3]dioxolan]-4-yl ((R)-1-phenylethyl)carbamate (M13S)

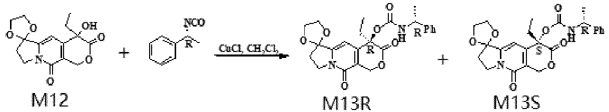

**M12** (5.5 g, 17.9 mmol) was dissolved in 200 mL anhydrous dichloromethane, and then (R)-α-phenyl-ethyl-isocyanate (5.26 g, 35.8 mmol) and cuprous chloride (3.45 g, 35.8 mmol) were added in sequence. Stir the mixture for 8 hrs at room temperature. Filter the inorganic salt and washed the residue using dichloromethane for three times. Then washed the solvent with water, and saturated salt water for three times.

Combined the organic layer and concentrated under reduced pressure to get yellow oil liquid. The mixture were purified by column chromatography (dichloromethane: ethyl acetate=4:1) to obtain two compounds **M13R** (3.92 g, 50%, [α]20D -9.36° (c 0.5, CHCl3); MS(ESI): m/z, 455.3 [M+H]+) and **M13S** (2.86 g, 45%, [α]20D +139° (c 0.5, CHCl3); MS(ESI): m/z, 455.1 [M+H]+).

¹H NMR (CDCl₃, 600 MHz) δ: 7.30 (m, 5 H, Ar-H), 6.10 (s, 1 H, Ar-H), 5.23, 5.55 (s, 2 H, ArCH₂O), 4.78 (m, 1 H, ArCH), 4.0-4.2 (m, 6 H, OCH₂CH₂O, NCH₂), 2.38 (t, 2 H, NCH₂CH₂), 1.95, 2.13 (s, 2 H, CH₂CH₃), 1.52 (d, 3 H, CHCH₃), 0.95 (t, 3 H, CH₃). ¹H NMR (CDCl₃, 600 MHz) δ: 7.31 (m, 5 H, Ar-H), 6.12 (s, 1 H, Ar-H), 5.20, 5.55 (s, 2 H, ArCH₂O), 4.71 (m, 1 H, ArCH), 3.9-4.1 (m, 6 H, OCH₂CH₂O, NCH₂), 2.38 (t, 2 H, NCH₂CH₂), 1.98, 2.17 (s, 2 H, CH₂CH₃), 1.55 (d, 3 H, CHCH₃), 0.95 (t, 3 H, CH₃).

### 6) Synthesis of (S)-4-ethyl-4-hydroxy-1,4,7,8-tetrahydro-3H,10H-spiro[pyrano[3,4-f]i ndolizine-6,2'-[1,3]dioxolane]-3,10-dione (M14)

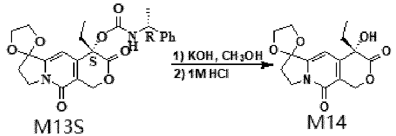

The mixture of M13S (1.1 g, 2.4 mmol) and potassium hydroxide (0.56 g, 9.9 mmol) were added in 25 mL methanol. The reaction was stirred in 67°C for 2 hrs and adjusted the pH to 2-3 using sulphuric acid. The mixture was extracted by dichloromethane for three times and combined the organic layer and concentrated under reduced pressure and purified by column chromatography (dichloromethane: ethyl acetate=4:1) to obtain **M14,** white solid, yield: 0.58 g (80%), mp 163-164°C; MS(ESI): m/z, 308.4 [M+H]⁺.

### 7) Synthesis of (S)-4-ethyl-4-hydroxy-7,8-dihydro-1H-pyrano[3,4-f]indolizine-3,6,10 (4H)-trione (M)

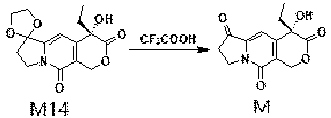

M14 (1 g, 3.26 mmol) was dissolved in 30 mL trifluroacetic acid and stirred for 6hrs at room temperature. The mixture was concentrated under reduced pressure and purified by column chromatography (dichloromethane: ethyl acetate=4:1) to get **M,** light yellow solid, yield: 0.88 g (93%), mp 183-185°C; [α]20D +101° (c 0.5, CHCl3); MS(ESI): m/z, 264.3 [M+H]⁺.

¹H NMR (CDCl₃, 600 MHz) δ: 6. 85 (s, 1 H, Ar-H), 5.34-5.42 (ABq, 2 H, ArCH₂O), 4.13 (m, 2 H, NCH₂), 2.89 (t, 2 H, NCH₂CH₂), 1.78 (m, 2 H, CH₂CH₃), 0.80 (t, 3 H, CH₃).

### Example 31 The anti-tumour activities of compounds in vitro

We tested compounds target a variety of tumor cell for the growth inhibition to show its antitumor activity by using the method of the field technicians known. Chooses the tumor cell lines include: A549 cell non-small cell lung cancer cell line (people), K562 cells (human chronic myeloid leukemia cells), NCI-H1975 cells (non-small cell lung adenocarcinoma cancer cell), MDA-MB-231 cells (human breast cancer cells) and HCT-116 cells (human colon cancer cells).

**Table 1 IC₅₀ values of the compounds for A549 and K562 cell lines**

| **compoun d** | **IC₅₀ (nM) A549** | **K562** | **compoun d** | **IC₅₀ (nM) A549** | **K562** |
|---|---|---|---|---|---|
| **Topoteca n** | 559 ± 8.6 | 636± 8.95 | **18** | 27± 3.25 | 489± 6.65 |
| **1** | 763± 5.05 | 1652± 10.61 | **20** | < 8 | 145± 4.35 |
| **2** | 1268± 4.65 | 2693± 9.63 | **32** | 48± 4.95 | 963± 7.61 |
| **3** | < 8 | 66 ± 3.65 | **33** | 174± 8.62 | 702± 8.87 |
| **6** | 530± 7.60 | 757± 8.62 | **36** | 422±10.35 | 1327± 8.96 |
| **7** | 1904± 8.64 | 1772± 9.68 | **37** | 387± 3.75 | 785± 9.23 |
| **9** | 1522± 10.5 | 1366± 12.51 | **38** | 282± 7.77 | 202± 7.32 |
| **10** | 1223± 8.75 | 564± 9.85 | **40** | 836± 8.69 | 1168± 9.69 |
| **11** | < 8 | 305± 875 | **41** | 50± 5 65 | 1037± 10.25 |
| **12** | 36± 4.65 | 1178± 13.55 | **42** | < 8 | 181± 8.55 |
| **14** | 19± 3.47 | 1949±15.65 | **43** | 13± 2.61 | 837±9.44 |
| **16** | < 8 | 115± 7.6 | **51** | < 8 | 13± 3.25 |

As shown in Table 1, most of the compounds in the invention showed better activity of cell proliferation compared to the drug topotecan. The compounds of **3, 11, 16, 20, 42, 43** and **51** have the IC₅₀ values between 1-10nM for the A549 cell line. The compounds of **12, 14, 18, 32, 41** and **43** have the IC₅₀ values between 10-50nM for the A549 cell line. Most of the target compounds showed better inhibition to A549 cells than to K562 cells, which meas the compounds in this invention has a good selectivity to cancer.

**Table 2. IC₅₀ values of the compounds for NCI-H1975, MDA-MB-231 and HCT-116 cell lines**

| **Compound** | IC₅₀ (nM) | | |
|---|---|---|---|
| | NCI-H1975 | MDA-MB-231 | HCT-116 |
| **21** | 20.36 ± 3.65 | 4.92 ± 0.36 | 0.74 ± 0.07 |
| **22** | 12.64 ±2.43 | 4.09 ± 0.46 | 0.18 ± 0.03 |
| **23** | 95.47 ±5.57 | 83.84 ± 6.64 | 2.97 ± 0.25 |
| **24** | 16.25 ± 0.83 | 1.93 ± 0.26 | 0.21 ± 0.02 |
| **25** | 35.20 ± 3.15 | 3.76 ± 1.08 | 0.34 ± 0.06 |
| **26** | 0.36 ± 0.06 | 0.56 ± 0.03 | 101.60 ± 6.18 |
| **27** | 4.53 ± 0.16 | 1.79 ± 0.14 | 0.25 ± 0.01 |
| **28** | 7.97 ± 0.52 | 1.56 ± 0.14 | 0.78 ± 0.06 |
| **29** | 79.73 ± 3.52 | 7.98 ± 0.72 | 1.84 ± 0.11 |
| **30** | 37.28 ± 2.82 | 43.41 ± 2.18 | 0.93 ± 0.08 |
| Topotecan | >500 | 123.40 ± 18.24 | >500 |

As shown in table 2, compounds **21-30** showed better activities of proliferation than the drug topotecan. The compound **26** had the best activities in NCI-H1975 and MDA-MB-231 cell lines. Compound **24** had the best cytotoxic activity for HCT-116 cell line. From the IC₅₀ values, the compounds **21-29** were most sensitive to HCT-116 cell line.

The above in vitro test showed that compounds of this invention have significant anti-tumor activity, and they can be used for the preparation of prevention or treatment of cancer drugs.

### Example 32 The compounds affect cell proliferation by experiment of macromolecular interaction combined with EGF, FGF .

The experimental scheme:
1. Fixed the compounds on the chip:

| topotecan | 1 | 2 | 3 | 4 | 5 | 6 |
|---|---|---|---|---|---|---|
| 7 | 8 | 9 | 10 | 51 | 52 | 32 |
| 14 | 21 | 23 | 24 | 25 | | |

| | | | | | | |
|---|---|---|---|---|---|---|
| Mobile phase were EGF (1µM), FGF (1µM), HSP90 (100nM), FKBP12 (100nM). Positive controls were RAPA and FKBP12. | | | | | | |

2. Measurements
Detection whether small molecule compounds combined with the provided protein of heat shock protein (HSP90), epidermal growth factor (EGF) and fibroblast growth factor (FGF); and provide relevant data of combined dynamics.
3. Results

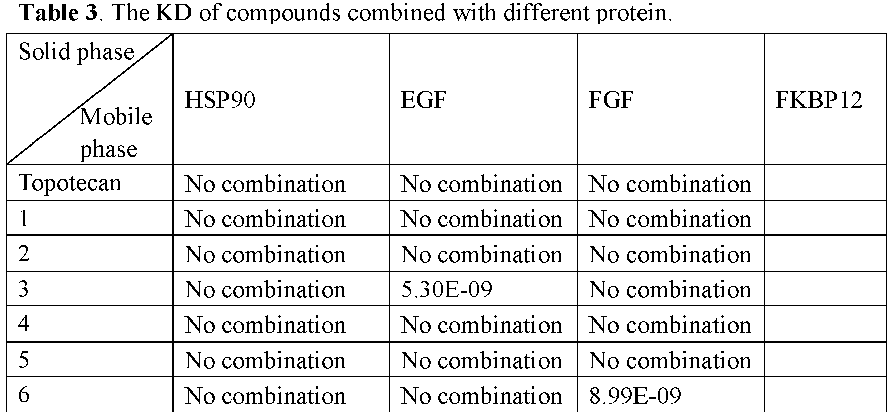

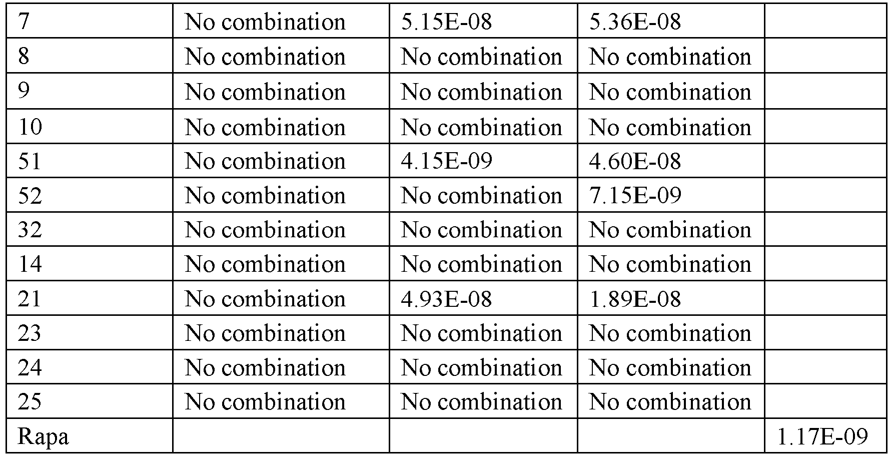

As shown in Table 3, the results showed that all the tested compounds have no combination with HSP90. Compounds **3, 7, 51** and **21** can combine with EGF and compounds **6, 7, 51, 52** and **21** can combine with FGF. Some compounds can combine with FGFs and EGF that have a relationship with cell proliferation, which means these compounds could target to the two proteins. Therefore, the compounds in this patent can be the inhibitor of FGF and EGF.

### Example 33 The anti-tumor activity in vivo

Using the known in the field of technology, the antitumor activity of compounds *in vivo* was tested in kunming mice transplanted tumor model. The transplantation tumor models tested including A549 lung transplantation tumor model and RM-1 (prostate) transplantation tumor model. We conducted the tests by intraperitoneal injection.

Compounds at low concentrations (9 mg/kg), which can effectively restrain the growth of the tumor (inhibitory rate reached more than 70%), at the same time showed much lower toxicity in mice and has the qualities of a medicine.

Compounds of **11, 15, 23,** and **51** with antitumor activity are better than that of topotecan. They have the qualities to be drugs.

### Example 34 The activity of anti-tumor of compound 23 in vivo

Using the known technology in the field, the inventor conducted antitumor activity *in vivo* of compound 23 by transplanting tumor in mice model.

### Methods including:

Recovering mice colon cancer cell lines HCT - 116 in liquid nitrogen, culturing with 5A medium containing 10% fetal bovine serum, expanding the cultured cells in the culture bottle. Digesting collecting cells after the desired amount of cells. Diluting the cells into 250 million/ml cell suspension with 5A culture medium containing 10% fetal bovine serum. Injecting 0.1 ml suspension to each mouse after routine disinfection in BALB/c-nu mice right forelimbs axillary region under the skin. Stay around tumor growth to 1 g tissue block, cutting short diameter growth of around 1 mm plaque, by inserting a catheter method. We began to treat medicine on the sixth day after transplantation According to the tumors size in mice, they were randomly divided into 7 groups, solvent control group, the stand for the Irinotecan group (6 mg/kg), 5 - fluorouracil group (20 mg/kg), compound 23 low dose group (2.5 mg/kg) and high dose group (5 mg/kg). Compound of low dose group and high dose group were given by gavages once a week, respectively. The group of irinotecan and 5 - fluorouracil were treated by intravenous every three days. Recording changes in body weight in mice, and tumors size three times a week.

### Results:

After subcutaneous inoculation of HCT-116 plaque in BALB/c-nu mice, the mice were treated medicine randomly when tumor volume went to 1.01 ± 0.65 mm³. In the 23rd day, when the tumor tissue of solvent control groups growth to 771.70 ± 101.05 mm³, put all the mice to death and kept all the tumor tissues - 80 °C.

Using days of treating medicine as the abscissa, drawing tumor volume growth curve. As shown in chart 2, compared with solvent control group and positive medicine group, low dose of and high dose of compound 23 can significantly inhibited the growth of HCT-116 transplanted tumors in mice. In the 23rd day after treating medicine, the inhibitory rates were 95.45% and 87.04%, respectively (Table 4).

**Table 4 Effects of compounds in the growth of the HCT-116 tumor in mice (x̅ ±SE)**

| Group | Dose (mg/kg) | Number (Before/After) | Tumor volume size (mm³) | Inhibitory rate (%) |
|---|---|---|---|---|
| **Solvent** | | 7/7 | 771.70±101.05 ^{a} | - |
| **Irinotecan** | 6 | 6/6 | 168.83±70.18 ^{a} | 78.12 ^{a} |
| **5** - **fluorouracil** | 20 | 6/6 | 342.28±128.00 ^{a} | 55.64 ^{a} |
| **Low dose of compound 23** | 2.5 | 6/6 | 100.52±20.45^{*a} | 87.04^{a} |
| **High dose of compound 23** | 5 | 5/6 | 19.94±2.17^{*a} | 95.45^{a} |

| | | | | |
|---|---|---|---|---|
| Ps: * p<0.01 vs the group of solvent; ^{a} measured after treating medicine for 23 days. | | | | |

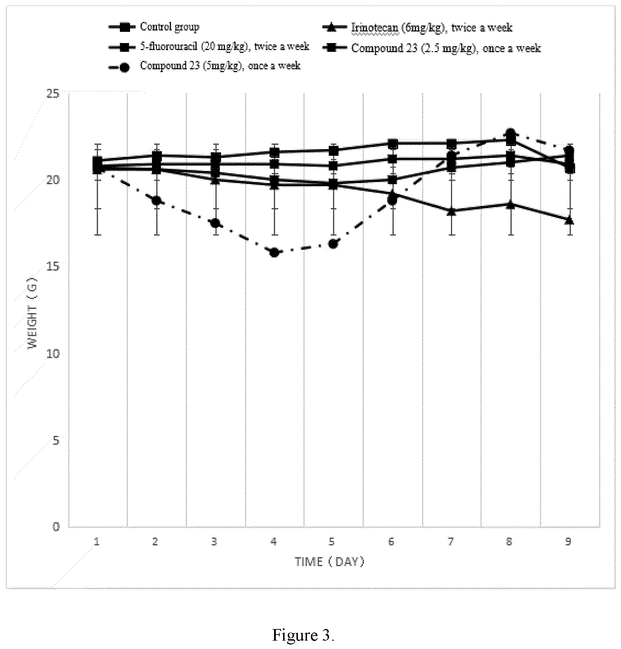

Using days of treating medicine as the abscissa, drawing the change of weight curve. As shown in Figure 3, The weight of solvent control group showed a trend of increased gradually. After the treatment of compound 23, mice weight in high and low dose group were declined. The weight returned after 10 days, but the weight still was lower than the control group, prompting its certain side effects. The mild side effects are common problem of camptothecin compounds.

## Claims

1. A compound represented by Formula I below, or a stereoisomer, a tautomer or a pharmaceutically acceptable salt thereof: 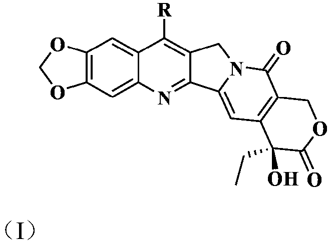 Wherein R is chosen among:

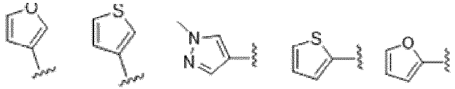

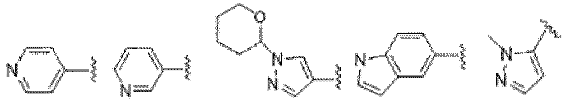

2. Compound according to claim 1, wherein the compound is chosen among the following compounds:

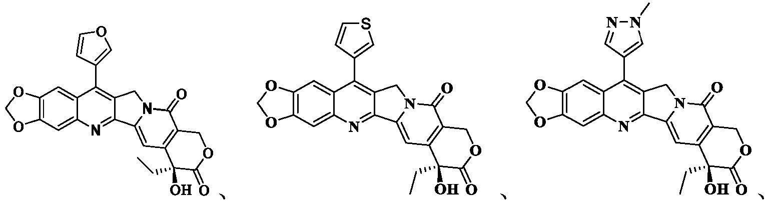

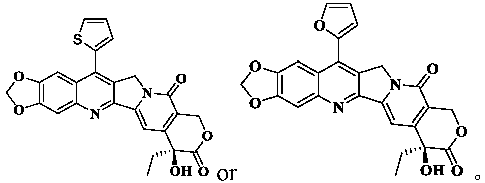

3. Compound according to any one of claims 1 to 2, for use in the prevention or treatment of cancer.

4. Compound according to any one of claims 1 to 2, for use according to claim 3, wherein the cancer is chosen from the group consisting of lung cancer, prostate cancer, colon cancer, leukemia and breast cancer.

5. A drug combination comprising:
1) an effective dose of a compound as defined in any one of claims 1-2, and
2) a pharmaceutically acceptable carrier.

## Patentansprüche

1. Verbindung, dargestellt durch die nachstehende Formel I, oder ein Stereoisomer, ein Tautomer oder ein pharmazeutisch akzeptables Salz davon 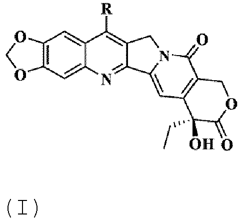 Wobei R ausgewählt wird aus

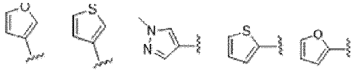

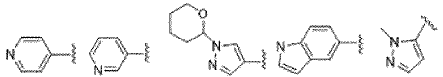

2. Verbindung nach Anspruch 1, wobei die Verbindung aus den folgenden Verbindungen ausgewählt ist:

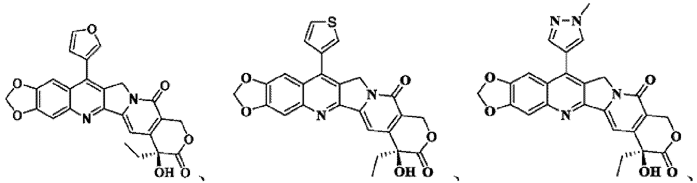

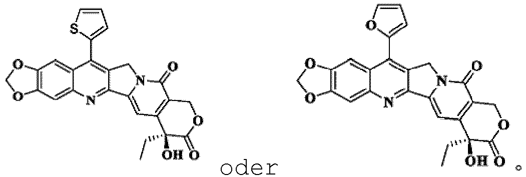

3. Verbindung nach einem der Ansprüche 1 bis 2 zur Verwendung bei der Prävention oder Behandlung von Krebs.

4. Verbindung nach einem der Ansprüche 1 bis 2, zur Verwendung nach Anspruch 3,
wobei der Krebs aus der Gruppe bestehend aus Lungenkrebs, Prostatakrebs, Dickdarmkrebs, Leukämie und Brustkrebs ausgewählt ist.

5. Arzneimittelkombination, umfassend:
1) eine wirksame Dosis einer Verbindung, wie sie in einem der Ansprüche 1-2 definiert ist, und
2) einen pharmazeutisch akzeptablen Träger.

## Revendications

1. Composé représenté par la formule I ci-dessous, ou un stéréo-isomère, un tautomère ou un sel pharmaceutiquement acceptable de celui-ci 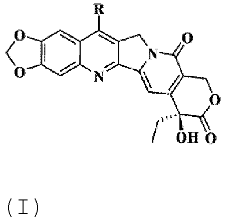 dans lequel R est choisi parmi

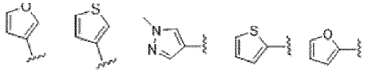

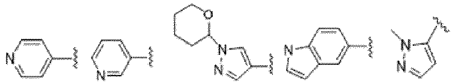

2. Composé selon la revendication 1, dans lequel le composé est choisi parmi les composés suivants:

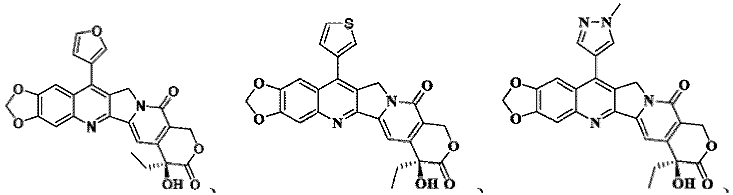

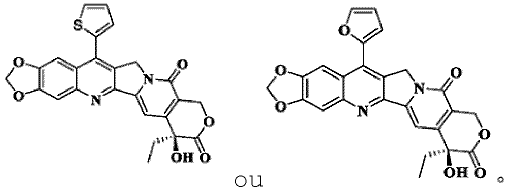

3. Composé selon l'une quelconque des revendications 1 à 2, pour utilisation dans la prévention ou le traitement du cancer.

4. Composé selon l'une quelconque des revendications 1 à 2, pour l'utilisation selon la revendication 3, dans lequel le cancer est choisi dans le groupe constitué par le cancer du poumon, le cancer de la prostate, le cancer du côlon, la leucémie et le cancer du sein.

5. Combinaison de médicaments comprenant:
1) une dose efficace d'un composé tel que défini dans l'une quelconque des revendications 1 à 2, et
2) un support pharmaceutiquement acceptable.
